# EUROPÄISCHE PATENTSCHRIFT

(11) **EP 2 224 982 B1**
(45) Veröffentlichungstag und Bekanntmachung des Hinweises auf die Patenterteilung: **03.07.2013**
(21) Anmeldenummer: 08862357.4
(22) Anmeldetag: 05.12.2008
(51) Int. Cl.: A61M 5/28, A61M 5/38

(54) **ZWEIKAMMER-SPRITZENVORRICHTUNG MIT GASDURCHLÄSSIGER MEMBRAN**
DUAL-CHAMBER INJECTION DEVICE HAVING GAS-PERMEABLE MEMBRANE
SYSTÈME DE SERINGUE À DEUX CHAMBRES COMPORTANT UNE MEMBRANE PERMÉABLE AUX GAZ

(30) Priorität: 19.12.2007 EP 07024613
(43) Veröffentlichungstag der Anmeldung: 08.09.2010
(73) Patentinhaber: Sanofi-Aventis Deutschland GmbH, 65929 Frankfurt am Main (DE)
(72) Erfinder: KÜHN, Bernd, 65926 Frankfurt am Main (DE)
(86) Internationale Anmeldenummer: PCT/EP2008/010318
(87) Internationale Veröffentlichungsnummer: WO 2009/077091

(56) Entgegenhaltungen:
- WO-A-2006/007592
- US-A- 4 226 236
- US-A- 4 373 535
- US-A- 5 114 421

## Beschreibung

Zweikammerspritzen werden unter anderem als Darreichungsform von medizinischen Präparaten entwickelt, wenn einzelne Komponenten des Präparates miteinander gemischt nur kurzzeitig für eine Anwendung stabil sind und daher für den Zeitraum einer längeren Lagerung voneinander separiert werden müssen. Verbreitetes Beispiel hierfür sind gefriergetrocknete Zubereitungen, bei denen hydrolyseempfindliche Wirkstoffe für den Lagerzeitraum vom Lösungsmittel separiert werden und erst unmittelbar vor der Anwendung zur Lösung rekonstituiert werden. Grundsätzlich gibt es dabei zwei Möglichkeiten die Komponenten zu kombinieren, zum einen die Kombination flüssig/flüssig und zum anderen die Kombination fest/flüssig. Zweikammer-Spritzensysteme haben den Vorteil, dass das Mischen der beiden Komponenten ohne Umfüllen in ein anderes Behältnis erfolgen kann und dass aus dem Behältnis heraus dann direkt die Verabreichung erfolgen kann. In Form von Zweikammer-Karpulen können die Behältnisse in dafür vorgesehene, gegebenenfalls mehrfach verwendbare Spritzengestelle oder Injektor-Systeme (Pen-Systeme oder Autoinjektor-Systeme) eingesetzt werden. Als Zweikammerspritze wird das Behältnis mit einem angeformten oder in den Kolben eingeschraubten Stößel oder einer Kolbenstange zum Vorschub der Kolben und mit oder ohne angesetzte Injektionsnadel bereitgestellt.

Für die Applikation, z.B. der parenteralen Anwendung, müssen Spritzenvorrichtungen wie beispielsweise Einwegspritzen oder Karpulen in der Regel zunächst entlüftet werden. Dieser Schritt ist insbesondere bei einer automatischen Abgabe aus Injektor-Systemen problematisch, da ein unkontrolliertes Austreten von hochpotenten pharmazeutischen Wirkstoffen vermieden werden muss.

US 4,226,236 beschreibt eine Zweikammer-Spritze. US 5,114,421 beschreibt einen Medikamentenbehälter.

US 5,971,953 beschreibt eine Zweikammerspritze mit einem unteren Kolben (lower piston), einem oberen Kolben und zylindrischem Schaft (tubular member), wobei Gas in der Mischkammer durch eine Membran im unteren Kolben und eine Öffnung im zylindrischen Schaft (opening) entweichen kann. Die in US 5,971,953 beschriebene Zweikammerspritze hat den Nachteil, dass zur Entlüftung eine Röhrenkonstruktion (Schaft) zur Ableitung der in der Spritze eingeschlossenen Luft durch den zweiten Kolben hindurch erforderlich ist. Der hierzu erforderliche zylindrische Schaft verlängert die Bauform der Spritze um mindestens die innerhalb des Spritzenzylinders zu verfahrende Weglänge. Eine Kombination mit Autoinjektoren führt dadurch zu unvorteilhaften Dimensionierungen die für die Marktfähigkeit problematisch sind. Weitere Nachteile liegen aufgrund der Vielzahl der einzelnen Komponenten in der hohen Komplexität von Fertigung und Montage der Zweikammerspritze.

DE 102004055870 beschreibt eine Einkammerspritze bestehend aus einem Zylinderelement und einer Kolbenvorrichtung, wobei in der Kolbenvorrichtung ein fluiddichtes, gasdurchlässiges Element angeordnet ist, durch das im Zylinderelement befindliches Gas beim Einführen der Kolbenvorrichtung in das Zylinderelement abgeführt werden kann.

EP 1237596 B1 beschreibt eine Einkammer-Spritzeneinheit bestehend aus einem Spritzenkörper mit Ausgabeende, einem innerhalb des Spritzenkörpers beweglich angeordneten Kolben und einem Verbindungsschlauch, der ein distales und ein erstes Ende aufweist, das mit dem Ausgabeende des Spritzenkörpers verbunden ist, wobei der Schlauch eine Entlüftungskappe mit einem Durchflußverhinderer aufweist, und wobei der Durchflußverhinderer gasdurchlässig, aber fluiddicht ist, und als Membran oder als Sperrventil (check valve) ausgeformt sein kann. Der Durchflußverhinderer dient zum Entlüften der Spritze nach dem Befüllen der Spritze und vor dem Verbinden des Verbindungsschlauches mit dem Patienten.

WO 2006007592 beschreibt ein Spritzensystem mit einer vorderen Kammer zur Aufnahme einer Flüssigkeit und einer hinteren luftgefüllten Kammer, wobei die hintere Kammer über eine Filtermembran und eine Öffnung steril belüftet werden kann.

US 4,373,535 beschreibt eine Einkammerspritze für die Entnahme von Blutproben bestehend aus einem zylindrischen Spritzenkörper und einer Kolbenvorrichtung, wobei die Kolbenvorrichtung eine gasdurchlässige, aber fluiddichte Membran enthält, die zur Entlüftung dient. Die in US 4,373,535 verwendete Membran besteht aus einem gasdurchlässigen Papier, das bei Kontakt mit Blut quillt und den weiteren Blutfluss stoppt. Wie in der in DE 102004055870 beschriebenen Einkammerspritze ist die Anordnung einer gasdurchlässigen Membran im Kolben nicht zweckmäßig für Doppelkammerspritzen, da hier die Lagerstabilität aufgrund von Gaspermeation zwischen den beiden Kammern und aus der hinteren Kammer in die Umgebung bei längerer Lagerung problematisch ist. Die Verwendung von Papiermembranen ist ferner nicht geeignet als Barriere zur Erhaltung eines sterilen Zustandes über die Lagerzeit.

Aufgabe der vorliegenden Erfindung ist es daher, eine verbesserte Spritzenvorrichtung bereit zu stellen, insbesondere eine Zweikammer-Spritzenvorrichtung, die ein vereinfachtes Entlüften erlaubt.

Die Erfindung betrifft eine Zweikammer-Spritzenvorrichtung wie sie in den Ausprüchen definert wird.

Verschlusselement (6) umfasst ferner optional ein Element zum Befestigen einer Nadel, einen Distanzring (10), der zwischen Dichtungselement und Zylinderelement (2) positioniert ist, und einen Dichtring (21), der zwischen Distanzring (10) und Zylinderelement (2) positioniert ist.

Die Zweikammer-Spritzenvorrichtung umfasst ferner optional eine Schutzkappe (14) zum Aufsatz auf das distale Ende des Zylinderelements (2) und/oder das distale Ende des Verschlusselementes (6).

Zwischen dem Verschlusselement (6) und dem distalen Kolben (3) wird innerhalb des Zylinderelements (2) eine Kammer A und zwischen dem distalen Kolben (3) und dem proximalen Kolben (4) eine Kammer B gebildet.

"Distal" bedeutet das Ende des jeweiligen Bauteils der Zweikammer-Spritzenvorrichtung, das im montierten Zustand zur Auslassöffnung (5a) weist. "Proximal" bedeutet das dem jeweiligen distalen Ende entgegengesetzte Ende.

Vorzugsweise ist die fluiddichte, gasdurchlässige Membran (8) am distalen (nadelseitigen) Ende des Zylinderelementes (2) im Bereich der Kammer A angeordnet. Ferner bevorzugt ist die fluiddichte, gasdurchlässige Membran (8) im Verschlusselement (6) angeordnet.

Die Zweikammer-Spritzenvorrichtung ist geeignet als Primärpackmittel für ein Medikament bestehend aus zwei Komponenten, wobei Kammer A eine flüssige oder eine feste Komponente, vorzugsweise eine feste Komponente, enthält und Kammer B eine flüssige Komponente. Enthält Kammer A eine feste Komponente, und Kammer B eine flüssige Komponente, ist die fluiddichte, gasdurchlässige Membran (8) insbesondere fluiddicht gegenüber der flüssigen Komponente im Gemisch mit der festen Komponente. Dieses Gemisch kann eine Lösung oder Dispersion (Emulsion oder Suspension) sein. Enthalten beide Kammern A und B eine flüssige Komponente, ist die fluiddichte, gasdurchlässige Membran (8) insbesondere fluiddicht gegenüber der flüssigen Komponente in Kammer A und gegenüber dem Gemisch beider flüssigen Komponenten. Die flüssige Komponente ist ein physiologisch verträgliches Lösungsmittel, beispielsweise Wasser oder eine wässrige Lösung wie z.B. ein wässriges Puffersystem. Die flüssige Komponente kann einen oder mehrere Wirkstoffe enthalten. Enthält Kammer A eine flüssige Komponente, so wird die fluiddichte, gasdurchlässige Membran (8) vorzugsweise durch eine gas-undurchlässige Schutzkappe (14) während der Lagerung abgedeckt, um Verluste an Flüssigkeit durch Gaspermeation durch die fluiddichte, gasdurchlässige Membran (8) auszuschließen oder wesentlich zu reduzieren.

Die fluiddichte, gasdurchlässige Membran (8) kann in unmittelbarem Produktkontakt stehen und ist somit Bestandteil des Primärpackmittels. Zur Vermeidung mikrobieller Verunreinigungen des enthaltenen Medikaments über die Lagerzeit werden vorzugsweise Sterilfiltermembranen verwendet. Besonders bevorzugt sind Sterilfiltermembranen mit einer nominellen Porengröße von kleiner gleich 0,2 µm, beispielsweise Sterilfiltermembranen aus hydrophobem Polytetrafluorethylen (PTFE). Die fluiddichte, gasdurchlässige Membran (8) ist eine hydrophobe Membran, die den Durchtritt von Gas, nicht jedoch den Durchtritt von wässrigen Flüssigkeiten wie die in der erfindungsgemäßen Zweikammer-Spritzenvorrichtung enthaltene flüssige Komponente erlaubt. Die Membranen sind durch geeignete Verfahren sterilisierbar (z.B. Strahlensterilisation, Ethylenoxid-Sterilisation), so dass sie als sterile Packmittelkomponente eingesetzt werden können. Gleichzeitig sind Membranen aus PTFE weitgehend inert, so dass sie eine gute Verträglichkeit mit einem weiten Spektrum verschiedener Produkte ermöglichen. Zur mechanischen Stabilisierung und Fixierung der Membran können, falls erforderlich, einseitig oder beidseitig Stützelemente, z.B. aus Polypropylen, aufgebracht werden (PPreinforced PTFE-membrane, nicht gezeichnet). Die Membran wird weiterhin für eine verbesserte mechanische Stabilität im Randbereich in einem Rahmen (8a) gehaltert. Die Herstellung solcher fixierter PTFE Membranen ist z.B. aus dem Bereich der Sterilfilterherstellung (Spritzenvorsatzfilter) bekannt.

Die erfindungsgemäße Zweikammer-Spritzenvorrichtung kann ferner ein Ventilelement (15) enthalten, das so über der gasdurchlässigen, fluiddichten Membran (8) angeordnet ist, dass ein Überdruck von Gas durch die Membran (8) und Ventilelement (15) passieren kann. Das Ventilelement (15) dient ferner als mechanischer Schutz der fluiddichten, gasdurchlässigen Membran (8) und als Barriere gegenüber dem Gasaustausch mit der Atmosphäre der Lagerumgebung des Produktes. Das Ventilelement (15) ist vorzugsweise als Ventilmembran ausgebildet.

In einer weiteren Ausführungsform sind die fluiddichte, gasdurchlässige Membran (8) und die Ventilmembran (15) oberhalb der distalen Öffnung (5a) angeordnet. In dieser Ausführungsform wird beim Aufsetzen einer Injektionsnadel die Ventilmembran und die fluiddichte, gasdurchlässige Membran (8) von der Nadel durchstoßen. Als Materialien für die Ventilmembran können beispielsweise Polyolefine (Polyethylen, Polypropylen, Polybutylen, Polyisobutylen) und Polyhalogenolefine (z.B. Polytetrafluorethylen (PTFE), Ethylen-Tetrafluorethlyen (ETFE)), Polyvinylchlorid (PVC), Ethyl-Vinyl-Acetat (EVA), Polystyrol (PS), Polyester (PES) (z.B. Polycarbonat (PC)) eingesetzt werden. Ebenso können natürliche und synthetische Elastomere wie Kautschuk, Halobutylkautschuk (Chlorbutylkautschuk, Brombutylkautschuk), EPDM und Silikonelastomere verwendet werden. Verschiedene Membranmaterialien können auch zur Einstellung bestimmter Eigenschaften (z.B. Wasserdampfpermeabilität, Gaspermeabilität) in Form von zwei- oder mehrschichtigen Laminaten miteinander kombiniert werden. Durch geeignete Einschnitte (15a) in der Membran, z.B. kreuzförmige Stanzöffnungen, wird eine Ventilfunktion hergestellt, da ein einseitig anstehender Überdruck eine Öffnung der Membran entlang der Schnittlinie in der Membran erzeugt und Überdruck dadurch entweichen kann. Im Ruhezustand stehen die Schnittkanten in der Membran geschlossen gegeneinander und bilden eine Barriere gegenüber einem ungehinderten atmosphärischen Austausch des Systems. Mit der Auswahl des Materials, den Membrandimensionen sowie der Form und Größe der Schnittlinien kann die erforderliche Druckdifferenz zur Ventilöffnung eingestellt werden. Ferner bevorzugt ist als Ventilelement (15) ein Einwegventil, das einen Gasdurchtritt nur aus der Spritzenvorrichtung heraus zulässt. Dies kann beispielsweise dadurch hergestellt werden, dass das Ventilelement unmittelbar auf der fluiddichten, gasdurchlässigen Membran (8) aufliegt und somit keine Ventilöffnung in Richtung der fluiddichten, gasdurchlässigen Membran (8) zulässt. In einer alternativen Ausführungsform kann das Ventilelement als einfaches Klappenventil hergestellt werden, indem die Ventilmembran auf einer umlaufenden Schulter aufliegt und dadurch eine Öffnung nur in einer Durchströmungsrichtung möglich ist.

Die Zweikammer-Spritzenvorrichtung enthält ferner mindestens einen Überleitungskanal (9), auch Bypass genannt, der es bei Verwendung der Zweikammer-Spritzenvorrichtung ermöglicht, dass sich eine in der proximalen Kammer enthaltene flüssige Komponente (17) während der Verabreichung des Medikaments unter Umgehung des Kolbens (3) bzw. der Kolben (3) und (3a) mit einer in der distalen Kammer enthaltenen festen oder flüssigen Komponente vermischen kann. Der Bypass kann erzeugt werden durch einen oder mehrere Kanäle, die sich im Wandmaterial des Zylinderelementes (2) befinden, d.h. in das Wandmaterial eingelassen bzw. eingearbeitet sind. Der Bypass kann ferner durch entsprechende Ausformung des Wandmaterials nach innen (nicht abgebildet) oder nach außen geformt werden. Die Anordnung kann axial oder von der axialen Richtung radial abweichend ausgeführt sein. Die Länge des Überleitungskanals ist größer als die Länge des Kolbens (3) bzw. bei Vorhandensein eines zusätzlichen Kolbens (3a) größer als die Summe der Längen der Kolben (3) und (3a), um ein Umfließen der Kolben zu gewährleisten.

Das Zylinderelement (2) kann beispielsweise aus Glas, Kunststoff, Metall oder anderen Werkstoffen geformt sein, vorzugsweise aus einem transparenten Werkstoff wie Glas oder Kunststoff. Bevorzugt entspricht das Glas der hydrolytischen Klasse 1 gemäß Europäischem Arzneibuch (Ph.Eur), welches klar transparent oder zur Erzielung eines Lichtschutzes eingefärbt sein kann. Die Herstellung des Zylinderelementes (2) aus Glas erfolgt bevorzugt aus Röhrenglas. Kunststoffe zur Formung des Zylinderelementes sind beispielsweise Polycarbonate, Polyester, Cyclo-Olefin-Copolymere (COC) oder Cyclic-Olefin-Polymere (COP). Vorzugsweise wird das Zylinderelement (2) aus Kunststoff im Reinraum spritzgegossen und danach magaziniert in hermetisch geschlossener Verpackung sterilisiert.

Das Verschlusselement (6) besteht zumindest aus einer Dichtscheibe (11) und einer Fixierhülse (12). Die Fixierhülse (12) bewirkt die dauerhafte gas- und fluiddichte Verbindung und die Dichtkraft zwischen Dichtungselement und dem Zylinderelement (2) und besteht beispielsweise aus Aluminium oder Kunststoff. Die Verbindung kann durch dem Fachmann bekannte Methoden, beispielsweise durch Crimpen, Bördeln, Aufprellen oder Verschrauben hergestellt werden. Als weiteres Bestandteil kann das Verschlusselement (6) ein Element zur Befestigung einer Nadel umfassen. Das Element zum Befestigen einer Nadel ist vorzugsweise ein Gewindeformteil (13), an das ein Nadelformteil aufgeschraubt werden kann. Alternativ kann ein Nadelformteil auf ein entsprechend ausgeformtes Element zum Befestigen einer Nadel aufgesteckt werden. Zusätzliche funktionelle Bestandteile, z.B. solche zur Fixierung der 2-Kammer-Spritzenvorrichtung in einem Pen oder Autoinjektor, können in das Verschlusselement integriert werden. Dies können z.B. Haken oder Laschen sein (nicht gezeichnet), die gegebenenfalls im Spritzgussverfahren an das Verschlussteil angeformt werden können. Weitere funktionelle erfindungsgemäße Bestandteile des Verschlusselements (6) können eine oder mehrere fluiddichte, gasdurchlässige Membranen (8) und Ventilmembranen (15) sein, *vide supra.* Ferner kann das Verschlusselement (6) einen Distanzring (10) enthalten und optional zusätzlich zum Distanzring (10) einen Dichtring (21).

Die Kolben (3), (3a) und (4), die Dichtscheibe (11) und der Dichtring (21) sind unabhängig voneinander aus elastischem Material, beispielsweise aus natürlichem oder synthetischem Kautschuk, vorzugsweise Brombutyl- oder Chlorbutyl-Kautschuk. Optional sind die Kolben mit PTFE beschichtet. Als weitere mögliche Ausführungsform können die Kolben aus thermoplastischen Kunststoffen (z.B. Polyethylen oder Polypropylen) gefertigt werden und deren fluiddichte Abdichtung gegen die Wand des Zylinderelementes (2) mittels angeformter Lamellen oder eingelegter Dichtringe (O-Ringe) aus elastischem Material wie oben beschrieben hergestellt werden. Die Kolben sind von vorzugsweise zylindrischer Grundform, aber auch andere Grundformen entsprechend der inneren Ausformung des Zylinderelements sind möglich. Die Kolben haben sowohl eine Dichtungs- als auch eine Verschlussfunktion. Die Dichtfunktion wird vorzugsweise durch eine oder mehrere lamellenartige Ausformungen der zylindrischen Grundform sichergestellt.

In einer weiteren Ausführungsform stellt die fluiddichte, gasdurchlässige Membran (8) das Dichtungselement dar, wobei die Zweikammer-Spritzenvorrichtung keine weitere Dichtscheibe (11) aufweist.

Der Distanzring (10) besteht vorzugsweise aus einem thermoplastischen Kunststoff (beispielsweise Polyethylen oder Polypropylen); seine fluiddichte Abdichtung gegen das Zylinderelement (2) wird gegebenenfalls mittels eingelegter Dichtringe (O-Ringe) (21) aus elastischem Material hergestellt. Der Distanzring dient als Bauteil, in das die Membran (8) und gegebenenfalls die Ventilmembran (15) eingefügt sind, und wird durch die Fixierhülse auf dem Zylinderelement (2) befestigt. Der Distanzring kann so ausgeformt sein, dass an seinem distalen Ende ein Element zum Befestigen einer Nadel vorliegt.

Die Verwendung von Kunststoff als Material für das Zylinderelement gewährleistet darüber hinaus die kostengünstige und präzise Herstellung der Teile, sowie die Integration von Funktionsteilen wie der Membran (8) oder des Verschlusselementes (6). Weiterhin können Funktionsteile, die für das Betreiben eines Pen- oder Autoinjektorsytems erforderlich sind, durch Spritzgussverfahren in einfacher Weise an das Zylinderelement (2) angeformt werden. Das Spritzgussverfahren stellt einen einfachen Prozess zur Herstellung dar, mit den Vorteilen leicht erreichbarer Partikelfreiheit, Pyrogenfreiheit, Sterilität, hoher Maßhaltigkeit und Recyclefähigkeit.

Das Zylinderelement (2) kann einteilig oder zweiteilig ausgeformt sein. In der einteiligen Ausführungsform besteht das Zylinderelement (2) aus einem Zylinder (2a). In der zweiteiligen Ausführungsform besteht das Zylinderelement (2) aus einem ersten, distalen Teilzylinder (2b) und einem zweiten, proximalen Teilzylinder (2c). In der zweiteiligen Ausführungsform kann der Überleitungskanal (9) im distalen oder im proximalen Teilzylinder positioniert sein.

In einer bevorzugten Ausführungsform eines zweiteiligen Zylinderelementes (2) ist mindestens einer, bevorzugt Teilzylinder (2b), besonders bevorzugt beide Teilzylinder (2b) und (2c) aus Kunststoff gefertigt. In einer besonders bevorzugten Ausführungsform sind beide Kunststoff-Bauteile über eine Schraubverbindung miteinander verbunden, wobei optional zur Abdichtung zwischen die Teilzylinder ein Dichtungselement eingefügt ist. Alternativ sind die Teilzylinder (2b) und (2c) bevorzugt über eine Steck- oder Klemmverbindung miteinander verbunden. Die Abdichtung der Teilzylinder kann durch Schweißtechniken (z.B. Hochfrequenz- oder Ultraschallschweißen) oder durch Verkleben mittels konventionellen pharmatauglichen Klebstoffen erfolgen, wobei optional zwischen den Teilzylindern (2b) und (2c) ein Dichtungselement eingefügt ist. In einer bevorzugten Ausführungsform ist der distale Teilzylinder (2b) bei der Herstellung aus Kunststoff so gefertigt, dass das funktionelle Bauteil der fluiddichten, gasdurchlässigen Membran (8) in den Bereich der Auslassöffnung (5a) integriert wird.

Ist der Überleitungskanal (9) im distalen Teilzylinder (2b) positioniert, bestehen beide Teilzylinder (2b) und (2c) bevorzugt aus Kunststoff und sind über eine Steck-, Klemm-oder Schraubverbindung miteinander verbunden, wobei optional zur Abdichtung zwischen die Teilzylinder ein Dichtungselement eingefügt ist. Der erste, distale Teilzylinder (2b) umfasst an seinem proximalen Ende einen Kolben (3). Die Auslassöffnung (5a) ist mit einem Verschlusselement (6) verschlossen. Speziell bevorzugt enthält in dieser Ausführungsform das distale Ende des zweiten, proximalen Teilzylinders (2c) eine axiale Nut (18), über die beim Zusammenbau der beiden Teilzylinder ein sich im Teilzylinder (2c) aufbauender Druck entweichen kann; alternativ kann der proximale Kolben (4) so im Teilzylinder (2c) positioniert werden, dass er beim Zusammenbau durch den aufgebauten Druck in die gewünschte Endlage am proximalen Ende verschoben wird. Optional enthält Teilzylinder (2c) am proximalen Ende einen Anschlag (19). Teilzylinder (2c) kann zusätzlich an seinem distalen Ende einen Kolben (3a) enthalten. Zwischen dem Verschlusselement (6) und dem distalen Kolben (3) wird innerhalb des Zylinderelements (2) eine Kammer A und zwischen Kolben (3a) und dem proximalen Kolben (4) eine Kammer B gebildet.

Die erfindungsgemäße zweiteilige Zweikammerkarpule hat den Vorteil, dass beide Teilzylinder getrennt voneinander durch den gesamten Durchmesser der Teilzylinder befüllbar sind, und dass der erste, distale Teilzylinder (2b) und bei Vorhandensein des Kolbens (3a) auch der zweite, proximale Teilzylinder (2c) getrennt voneinander produzierbar und somit lagerfähig sind. Der optionale Zwischenkolben (3a) bewirkt außerdem, dass keine Kontaminationsgefahr durch feste oder flüssige Komponente an den Kontaktstellen zwischen erstem und zweitem Teilzylinder besteht. Vorteil der zweiteiligen Ausführungsform ist ferner ein maximaler Öffnungsdurchmesser, entsprechend dem gesamten Innendurchmesser des Zylinders, zur Befüllung mit festen oder flüssigen Komponenten, wodurch der Herstellschritt der Befüllung insbesondere bei schlecht fließfähigen Pulvern vorteilhaft durchgeführt werden kann. Durch die Möglichkeit der direkten Befüllung über große Öffnungen ist kein Lyophilisieren der festen Komponente in Kammer A mehr notwendig. Stattdessen kann die feste Komponente, vorzugsweise in Pulverform, eingefüllt werden. Die schonende Pulverbefüllung gewährleistet ferner, dass keine Beeinflussung der morphologischen Struktur des Pulvers auftritt. Die Karpule zeichnet sich ferner durch hervorragende Wirtschaftlichkeit aus, da durch Schnelligkeit der Befüllung und gegebenenfalls Anpassung der Geschwindigkeit der Befüllung des distalen Teilzylinders (2b) an die der Befüllung des proximalen Teilzylinders (2c) das Verfahren zur Herstellung und Befüllung der Zweikammerkarpule vorteilhaft ist.

Die Zweikammer-Spritzenvorrichtung ist als Karpule ausgebildet. Die Zweikammerkarpule ist in Applikations-Systeme, beispielsweise Pen- oder Autoinjektorsysteme einsetzbar, wobei das Applikations-System ferner eine Nadel zum Durchstoßen des Dichtungselements (11) enthält, sowie einen Antriebsmechanismus zum Bewegen des Kolbens (4) in distaler Richtung.

In einer weiteren Ausführungsform enthält die Zweikammer-Spritzenvorrichtung zusätzlich eine am distalen Ende des Zylinderelements befestigte Nadel, die dafür vorgesehen ist, die Dichtscheibe (11) zu durchstechen, und eine Vorrichtung zum Bewegen des Kolbens (4) in distaler Richtung. Beispielsweise ist eine solche Zweikammer-Spritzenvorrichtung als Einwegspritze ausgebildet.

"Medikament" bedeutet eine pharmazeutische Formulierung enthaltend mindestens eine wirksame niedermolekulare Verbindung mit einem Molekülgewicht bis zu 1500 Da, ein pharmazeutisches wirksames Peptid, Protein, DNA, RNA, Antikörper, Enzym, Hormon oder Oligonukleotid, oder ein Gemisch davon,
vorzugsweise enthaltend mindestens ein Peptid, bevorzugt ein Peptid zur Behandlung von Diabetis mellitus oder Komplikationen von Diabetis mellitus wie bespielsweise diabetische Retinopathie,
besonders bevorzugt Humaninsulin oder ein Humaninsulinanalogon oder -derivat, Glucagon-Like Peptide-1 (GLP1) oder ein Analogon oder Derivat davon, oder Exendin-3 oder Exendin-4 oder ein Analogon oder Derivate von Exendin-3 oder Exendin-4.

Insulinanaloga sind beispielsweise Gly(A21), Arg(B31), Arg(B32)- Humaninsulin; Lys(B3), Glu(B29)- Humaninsulin; Lys(B28), Pro(B29)- Humaninsulin; Asp(B28)- Humaninsulin; Humaninsulin, bei dem Prolin in der Position B28 substituiert wurde durch Asp, Lys, Leu, Val oder Ala und wo in Position B29 Lys durch Pro substituiert sein kann; Ala(B26)-Humaninsulin; Des(B28-B30)- Humaninsulin; Des(B27)- Humaninsulin und Des(B30)-Humaninsulin.

Insulinderivate sind beispielsweise B29-N-myristoyl-des(B30) Humaninsulin; B29-N-palmitoyl-des(B30) Humaninsulin; B29-N-myristoyl Humaninsulin; B29-N-palmitoyl Humaninsulin; B28-N-myristoyl LysB28ProB29 Humaninsulin; B28-N-palmitoyl-LysB28ProB29 Humaninsulin; B30-N-myristoyl-ThrB29LysB30 Humaninsulin; B30-N-palmitoyl- ThrB29LysB30 Humaninsulin; B29-N-(N-palmitoyl-Y-glutamyl)-des(B30) Humaninsulin; B29-N-(N-lithocholyl-Y-glutamyl)-des(B30) Humaninsulin; B29-N-(*ω-*carboxyheptadecanoyl)-des(B30) Humaninsulin und B29-N-(*ω*-carboxyheptadecanoyl) Humaninsulin.

Exendin-4 bedeutet vorzugsweise Exendin-4(1-39), ein Peptid mit der Sequenz H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH₂.

Exendin-4-Derivate sind beispielsweise ausgewählt aus der folgenden Gruppe von Verbindungen:
H-(Lys)₄-des Pro³⁶, des Pro³⁷ Exendin-4(1-39)-NH₂,
H-(Lys)₅-des Pro³⁶, des Pro³⁷ Exendin-4(1-39)-NH₂,
des Pro³⁶ [Asp²⁸] Exendin-4(1-39),
des Pro³⁶ [IsoAsp²⁸] Exendin-4(1-39),
des Pro³⁶ [Met(O)¹⁴, Asp²⁸] Exendin-4(1-39),
des Pro³⁶ [Met(O)¹⁴, IsoAsp²⁸] Exendin-4(1-39),
des Pro³⁶ [Trp(O₂)²⁵, Asp²⁸] Exendin-4(1-39),
des Pro³⁶ [Trp(O₂)²⁵, IsoAsp²⁸] Exendin-4(1-39),
des Pro³⁶ [Met(O)¹⁴ Trp(O₂)²⁵, Asp²⁸] Exendin-4(1-39),
des Pro³⁶ [Met(O)¹⁴ Trp(O₂)²⁵, IsoAsp²⁸] Exendin-4(1-39); oder

des Pro³⁶ [Asp²⁸] Exendin-4(1-39),
des Pro³⁶ [IsoAsp²⁸] Exendin-4(1-39),
des Pro³⁶ [Met(O)¹⁴, Asp²⁸] Exendin-4(1-39),
des Pro³⁶ [Met(O)¹⁴, IsoAsp²⁸] Exendin-4(1-39),
des Pro³⁶ [Trp(O₂)²⁵, Asp²⁸] Exendin-4(1-39),
des Pro³⁶ [Trp(O₂)²⁵, IsoAsp²⁸] Exendin-4(1-39),
des Pro³⁶ [Met(O)¹⁴ Trp(O₂)²⁵, Asp²⁸] Exendin-4(1-39),
des Pro³⁶ [Met(O)¹⁴ Trp(O₂)²⁵, IsoAsp²⁸] Exendin-4(1-39),
wobei die Gruppe -Lys₆-NH₂ mit dem C-Terminus des Exendin-4-Derivats verknüpft ist; oder
ein Exendin-4-Derivat der Sequenz
H-(Lys)₆-des Pro³⁶ [Asp²⁸] Exendin-4(1-39)-Lys₆-NH₂,
des Asp²⁸ Pro³⁶, Pro³⁷, Pro38Exendin-4(1-39)-NH₂,
H-(Lys)₆-des Pro³⁶, Pro³⁸ [Asp²⁸] Exendin-4(1-39)-NH₂,
H-Asn-(Glu)₅des Pro³⁶, Pro³⁷, Pro³⁸ [Asp²⁸] Exendin-4(1-39)-NH₂,
des Pro³⁶, Pro³⁷, Pro³⁸ [Asp²⁸] Exendin-4(1-39)-(Lys)₆-NH₂,
H-(Lys)₆-des Pro³⁶, Pro³⁷, Pro³⁸ [Asp²⁸] Exendin-4(1-39)-(Lys)₆-NH₂,
H-Asn-(Glu)₅-des Pro³⁶, Pro³⁷, Pro³⁸ [Asp²⁸] Exendin-4(1-39)-(Lys)₆-NH₂,
H-(Lys)₆-des Pro³⁶ [Trp(O₂)²⁵, Asp²⁸] Exendin-4(1-39)-Lys₆-NH₂,
H-des Asp²⁸ Pro³⁶, Pro³⁷, Pro³⁸ [Trp(O₂)²⁵] Exendin-4(1-39)-NH₂,
H-(Lys)₆-des Pro³⁶, Pro³⁷, Pro³⁸ [Trp(O₂)²⁵, Asp²⁸] Exendin-4(1-39)-NH₂,
H-Asn-(Glu)₅-des Pro³⁶, Pro³⁷, Pro³⁸ [Trp(O₂)²⁵, Asp²⁸] Exendin-4(1-39)-NH₂,
des Pro³⁶, Pro³⁷, Pro³⁸ [Trp(O₂)²⁵, Asp²⁸] Exendin-4(1-39)-(Lys)₆-NH₂,
H-(Lys)₆-des Pro³⁶, Pro³⁷, Pro³⁸ [Trp(O₂)²⁵, Asp²⁸] Exendin-4(1-39)-(Lys)₆-NH₂,
H-Asn-(Glu)₅-des Pro³⁶, Pro³⁷, Pro³⁸ [Trp(O₂)²⁵, Asp²⁸] Exendin-4(1-39)-(Lys)₆-NH₂,
H-(Lys)₆-des Pro³⁶ [Met(O)¹⁴, Asp²⁸] Exendin-4(1-39)-Lys₆-NH₂,
des Met(O)¹⁴ Asp²⁸ Pro³⁶, Pro³⁷, Pro³⁸ Exendin-4(1-39)-NH₂,
H-(Lys)₆-desPro³⁶, Pro³⁷, Pro³⁸ [Met(O)¹⁴, Asp²⁸] Exendin-4(1-39)-NH₂,
H-Asn-(Glu)₅-des Pro³⁶, Pro³⁷, Pro³⁸ [Met(O)¹⁴, Asp²⁸] Exendin-4(1-39)-NH₂,
des Pro³⁶, Pro³⁷, Pro³⁸ [Met(O)¹⁴, Asp²⁸] Exendin-4(1-39)-(Lys)₆-NH₂,
H-(Lys)₆-des Pro³⁶, Pro³⁷, Pro³⁸ [Met(O)¹⁴, Asp²⁸] Exendin-4(1-39)-(Lys)₆-NH₂,
H-Asn-(Glu)₅ des Pro³⁶, Pro³⁷, Pro³⁸ [Met(O)¹⁴, Asp²⁸] Exendin-4(1-39)-(Lys)₆-NH₂,
H-Lys₆-des Pro³⁶ [Met(O)¹⁴, Trp(O₂)²⁵, Asp²⁸] Exendin-4(1-39)-Lys₆-NH₂,
H-des Asp²⁸ Pro³⁶, Pro³⁷, Pro³⁸ [Met(O)¹⁴, Trp(O₂)²⁵] Exendin-4(1-39)-NH₂,
H-(Lys)₆-des Pro³⁶, Pro³⁷, Pro³⁸ [Met(O)¹⁴, Asp²⁸] Exendin-4(1-39)-NH₂,
H-Asn-(Glu)₅-des Pro³⁶, Pro³⁷, Pro³⁸ [Met(O)¹⁴, Trp(O₂)²⁵, Asp²⁸] Exendin-4(1-39)-NH₂,
des Pro³⁶, Pro³⁷, Pro³⁸ [Met(O)¹⁴, Trp(O₂)²⁵, Asp²⁸] Exendin-4(1-39)-(Lys)₆-NH₂,
H-(Lys)₆-des Pro³⁶, Pro³⁷, Pro³⁸ [Met(O)¹⁴, Trp(O₂)²⁵, Asp²⁸] Exendin-4(S1-39)-(Lys)₆-NH₂,
H-Asn-(Glu)₅-des Pro³⁶, Pro³⁷, Pro³⁸ [Met(O)¹⁴, Trp(O₂)²⁵, Asp²⁸] Exendin-4(1-39)-(Lys)₆-NH₂;
oder ein pharmazeutisch akzeptables Salz oder Solvat eines der vorgenannten Exendin-4-Derivate.

Hormone sind vorzugsweise Hypophysenhormone oder Hypothalamushormone oder regulatorisch aktive Peptides, sowie Antagonisten davon entsprechend der Publikation Rote Liste, Ausgabe 2008, Kapitel 50. Beispiele für Hormone sind Gonadotropin (Follitropin, Lutropin, Choriongonadotropin, Menotropin), Somatropin, Desmopressin, Terlipressin, Gonadorelin, Triptorelin, Leuprorelin, Buserelin, Nafarelin, Goserelin.

Pharmazeutisch akzeptable Salze sind beispielsweise Säureadditionssalze und basische Salze. Säureadditionssalze sind beispielsweise HCl- oder HBr-Additionssalze. Basische Salze sind beispielsweise Salze, in denen das Kation ausgewählt ist aus der Gruppe der Alkalisalze, beispielsweise Na⁺ oder K⁺, oder der Erdalkalisalze, beispielsweise Ca²⁺, oder Ammoniumionen N⁺(R₁)(R₂)(R₃)(R₄), wobei R₁ bis R₄ unabhängig voneinander bedeuten: Wasserstoff, optional substituiertes C₁-C₆-Alkyl, optional substituiertes C₂-C₆-Alkenyl, optional substituiertes C₆-C₁₀-Aryl, oder optional substituiertes C₆-C₁₀-Heteroaryl. Weitere Beispiele von pharmazeutisch akzeptablen Salze sind beschrieben in "Remington's Pharmaceutical Sciences" 17. Ed. Alfonso R. Gennaro (Ed.), Mark Publishing Company, Easton, Pa., U.S.A., 1985 und in Encyclopedia of Pharmaceutical Technology.

Pharmazeutisch akzeptable Solvate sind z.B. Hydrate.

Allen Ausführungsformen ist gemein, dass die flüssige Komponente und die feste Komponente bevorzugt unter aseptischen Bedingungen abgefüllt werden. Bei ausreichender Stabilität des Produktes kann gegebenenfalls zur zusätzlichen Sicherstellung der Sterilität eine Behandlung mit ionisierenden Strahlen angeschlossen werden.

Die Zweikammer-Spritzenvorrichtung kann ferner zur Vermeidung von Umwelteinflüssen und unbeabsichtigter Verunreinigung während der Lagerung umverpackt werden. Falls zur Erzielung ausreichender Lagerstabilität erforderlich, kann die Umverpackung bevorzugt aus Folienmaterialien hergestellt werden, die eine Gasbarriere darstellen. Solche gasdichten Folienmaterialien sind beispielsweise Aluminiumfolien, kunststofflaminierte Aluminiumfolien oder aluminiumbeschichtete Kunststofffolien. Weitere geeignete Folien sind Kunststofffolien aus Monomaterialien oder laminierte Kunststofffolien aus zwei oder mehreren Schichten, die ebenfalls in Abhängigkeit ihrer Foliendicke und Zusammensetzung gute Gasbarrieren darstellen können. Die hierfür in Frage kommenden Folienmaterialien sind dem Fachmann der Verpackungstechnik bekannt und über deren Kennzahlen zur Gas- und Wasserdampfdurchlässigkeit charakterisiert.

Für die erfindungsgemäße Verwendung können dem Anwender als kit-of-parts alle benötigten Komponenten in einer Packung zur Verfügung gestellt werden. Dies beinhaltet neben der Zweikammer-Spritzenvorrichtung als Karpule, Einwegspritze, Pen oder Autoinjektor zusätzlich Nadeln zum Aufsetzen, sowie optional ein oder mehrere Desinfektions-Tupfer zur Desinfektion der Oberfläche des Verschlusselements (6) vor Aufsetzen der Nadel und zur Desinfektion der Einstichstelle der Haut.

Die erfindungsgemäße Zweikammer-Spritzenvorrichtung weist den Vorteil auf, dass sie eine einfache und sehr kostengünstige Lösung bereitstellt, um ein in der Spritzenvorrichtung befindliches gasförmiges Medium aus dem Spritzenzylinder zu entfernen. Ein Entlüften mittels einer Nadel wird vermieden. Der Patient setzt sich somit nicht mehr der Gefahr aus, eine Nadel auf ein unter Druck stehendes System setzen zu müssen und sich dabei mit einem hochpotenten Medikament ungewollt zu kontaminieren. Bei Verwendung des Applikationssystems über einen längeren Zeitraum oder bei Lagerung über einen langen Zeitraum kann sich Gas, das bisher in der flüssigen Komponente gelöst war, beispielsweise durch Erwärmung des Medikaments von der flüssigen Komponente trennen. Dies macht gegebenenfalls ein erneutes Entlüften notwendig, was durch das erfindungsgemäße Zweikammer-Spritzensystem vereinfacht wird.

Die erfindungsgemäße Zweikammer-Spritzenvorrichtung lässt sich ferner in automatischen Applikationsgeräten verwenden, wobei der Entlüftungsschritt ebenfalls automatisch erfolgen kann.

Die erfindungsgemäße Zweikammer-Spritzenvorrichtung kann beispielsweise wie folgt verwendet werden: Zunächst wird in einem Mischungsschritt der Kolben (4) in distaler Richtung bewegt, wodurch unter gleichzeitigem Vorschub des Kolbens (3) der Inhalt der Kammer B über den Bypass (9) in die Kammer A überführt und gemischt wird. Dabei kann in der Zweikammer-Spritzenvorrichtung enthaltenes Gas durch die fluiddichte, gasdurchlässige Membran (8) bereits entweichen, da sich durch den Vorschub der Kolben (3) und (4) das Volumen innerhalb der Zweikammer-Spritzenvorrichtung verringert und somit durch Komprimieren des Gases ein Überdruck im System entsteht. Beim nachfolgenden Entlüftungsschritt (priming) werden die Kolben (3) und (4) gemeinsam in distaler Richtung bewegt, wobei ein Druck in der Vorrichtung aufgebaut wird. Der Druck kann vor dem Aufsetzen einer Nadel aus dem System entweichen, sobald die Orientierung der fluiddichten, gasdurchlässigen Membran (8) mit der Orientierung des Gases in Kammer A übereinstimmt. Ist beispielsweise die Dichtscheibe (11) als die fluiddichte, gasdurchlässige Membran (8) ausgebildet, kann Gas entweichen, wenn die Zweikammer-Spritzenvorrichtung aufrecht (mit dem Verschlusselement nach oben) ausgerichtet wird. Die Orientierung der Spritzenvorrichtung muss nicht notwendigerweise bereits beim Bewegen des Kolbens (4) in distaler Richtung korrekt sein, jedoch soll die Entlüftung vor dem Aufsetzen der Nadel erfolgt sein. Beim Aufsetzen der Nadel, bei dem das Dichtungselement durchstochen wird, enthält die Zweikammer-Spritzenvorrichtung dann kein komprimiertes Gas mehr. Sofern durch den Kolben kein weiterer Vorschubdruck erzeugt wird besteht also keine Gefahr, dass der Anwender durch unkontrolliert austretendes Medikament kontaminiert wird oder Medikament verlorengeht.

Generell ist eine Kombination aller der genannten allgemeinen und bevorzugten Merkmale der Ausführungsformen technisch möglich.

Weitere Ausgestaltungen und Vorteile der Erfindung ergeben sich aus der folgenden Beschreibung der in den Zeichnungen dargestellten Ausführungsbeispiele.

### Beispiel 1

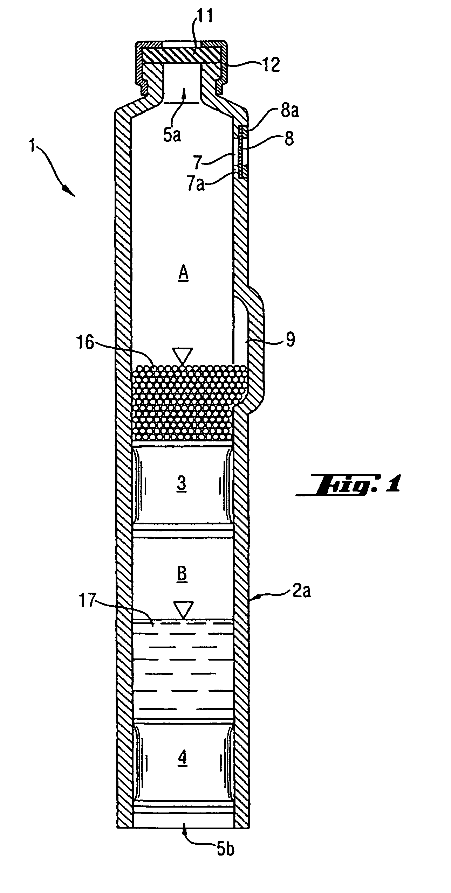

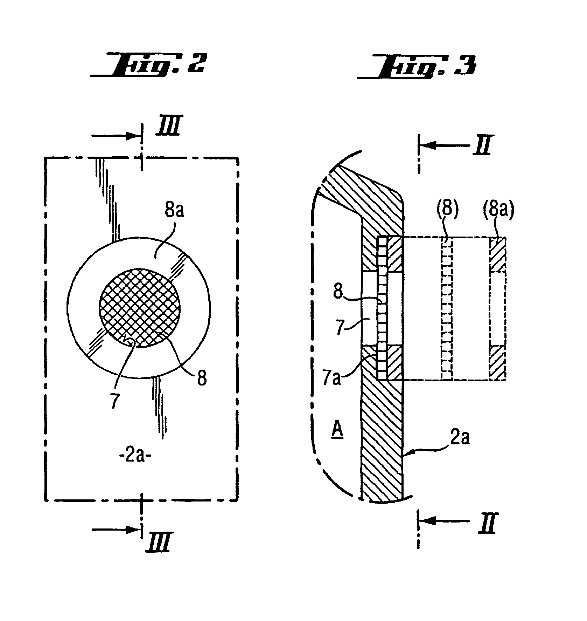

Nachfolgend wird unter Bezugnahme auf die Fig. 1 bis 3 eine Zweikammer-Spritzenvorrichtung (1) gemäß einem ersten Ausführungsbeispiel der Erfindung beschrieben.

Wie in Fig. 1 gezeigt, umfasst die Zweikammer-Spritzenvorrichtung (1) ein Zylinderelement (2) bestehend aus einem Zylinder (2a), zwei Kolben (3) und (4), sowie ein Verschlusselement (6, Ziffer nicht abgebildet). Das Zylinderelement (2) weist eine Auslassöffnung (5a) und eine große Öffnung (5b) am proximalen Ende auf, in welche die Kolben (3) und (4) eingeführt werden. Die Auslassöffnung (5a) ist mit einem Verschlusselement (6) verschlossen. Zwischen dem Verschlusselement (6) und dem Kolben (3) und begrenzt durch die Wand des Zylinders (2a) entsteht eine Kammer A. Zwischen dem Kolben (3) und dem Kolben (4) und begrenzt durch die Wand des Zylinders (2a) entsteht eine Kammer B. In der Wand des Zylinders (2a) im Bereich der Kammer A unterhalb der Schulter am distalen Ende ist eine Bohrung (7) vorgesehen. Die Bohrung (7) ist durch eine fluiddichte, gasdurchlässige Membran (8) verschlossen. Fertigungstechnisch ist dies beispielsweise möglich, indem die Wandung im Bereich der Bohrung (7) ein Widerlager oder eine Schulter (7a) bildet, auf die die Membran aufgelegt und mittels eines Rahmens (8a) verschweißt wird. In die Wand des Zylinders (2a) ist ferner im Bereich der Kammer A und nahe dem distalen Ende des Kolbens (3) eine Überleitung (9) eingeformt. Das Verschlusselement (6) besteht in diesem Beispiel aus einer Dichtscheibe (11) und einer Fixierhülse (12). Die Dichtscheibe (11) ist auf die Auslassöffnung (5a) aufgesetzt und wird durch die Fixierhülse (12) umschlossen und durch Verbördeln mit dem Zylinder (2a) fest verbunden.

Die Montage, das Befüllen und die Anwendung der erfindungsgemäßen Zweikammer-Spritzenvorrichtung (1) ist dabei wie folgt: Zunächst wird der Kolben (3) durch die hintere Öffnung (5b) in den Zylinder (2a) eingeführt und positioniert. Dann wird durch die Öffnung (5b) die Kammer B mit dem flüssigen Medium gefüllt. Danach wird der Kolben (4) in die Öffnung (5b) eingeführt und die Kammer B damit verschlossen. Die Bauteile werden um 180 Grad gedreht und Kammer A wird durch die Auslassöffnung (5a) mit einem festen oder flüssigen Medium gefüllt. Danach wird die Auslassöffnung (5a) mit dem Verschlusselement (6) verschlossen. Zur Vorbereitung der Anwendung wird der Kolben (4) durch eine angesetzte Kolbenstange (nicht abgebildet) in Richtung der Auslassöffnung (5a) geschoben. Da das Fluid in Kammer B inkompressibel ist, wird gleichzeitig der Kolben (3) in eine Vorschubbewegung versetzt. Hierbei kann die in Kammer A befindliche Luft durch die fluiddichte, gasdurchlässige Membran (8) entweichen, so dass in Kammer A kein Überdruck entsteht. Die Zweikammer-Spritzenvorrichtung wird dabei in annähernd horizontaler Lage so gehalten, dass die Bohrung (7) an höchster Position ist. Wenn durch die Vorschubbewegung der Kolben (3) den Bereich der eingeformten Überleitung (9) erreicht hat, kann das Fluid aus Kammer B durch die Überleitung (9) in die Kammer A eintreten und sich mit dem festen oder flüssigen Medium in Kammer A mischen. Dabei kann eingeschlossene Luft über die Membran (8) entweichen. Wenn Kolben (4) in Kontakt mit Kolben (3) ist, ist die Überleitung des Mediums aus Kammer B beendet und die Inhalte der Kammern A und B können homogen gemischt werden. Anschließend werden beide Kolben (3) und (4) gemeinsam weiter vorgeschoben bis die Luft aus der Kammer A über die Membran (8) verdrängt ist. Da die Membran (8) nur für Gas durchlässig ist, nicht jedoch für das Fluid, ist dann kein weiterer Vorschub der Kolben (3) und (4) mehr möglich. Die Zweikammer-Spritzenvorrichtung ist dann für die Applikation des Mediums vorbereitet.

Für die Applikation wird dann in herkömmlicher Weise eine Applikationsnadel auf das Verschlusselement (6) aufgesetzt und die Dichtscheibe (11) durchstochen.

### Beispiel 2

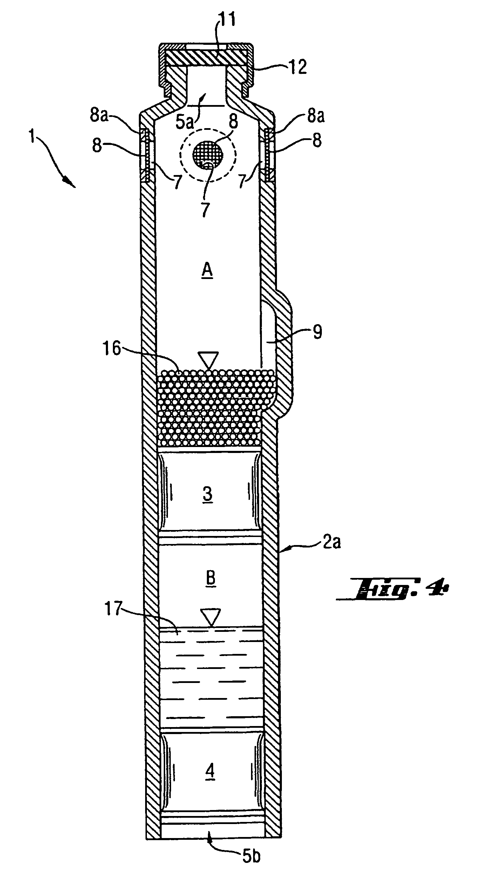

Nachfolgend wird unter Bezugnahme auf Fig. 4 eine Zweikammer-Spritzenvorrichtung (1) gemäß einem zweiten Ausführungsbeispiel der Erfindung beschrieben. Gleiche Teile sind dabei mit den gleichen Bezugszeichen wie im ersten Ausführungsbeispiel bezeichnet. Wie in der schematischen Ansicht von Fig. 4 ersichtlich ist, umfasst die Zweikammer-Spritzenvorrichtung (1) ein Zylinderelement (2) bestehend aus einem Zylinder (2a), zwei Kolben (3) und (4), sowie ein Verschlusselement (6, Ziffer nicht abgebildet). Im Zylinderelement (2) sind im Unterschied zum ersten Ausführungsbeispiel im Bereich der Kammer A unterhalb der Schulter am distalen Ende vier Bohrungen (7) auf dem Umfang des Zylinders (2a) ausgebildet, welche jeweils mit einer fluiddichten, gasdurchlässigen Membran (8) verschlossen sind. Hierdurch kann die verschlossene Zweikammer-Spritzenvorrichtung in weitgehend horizontaler Lage und nahezu unabhängig von einer Ausrichtung des Zylinderelements (2) entlüftet werden, da mehrere mit gasdurchlässiger Membran (8) verschlossene Öffnungen (7) vorhanden sind und das Gas in der Zweikammer-Spritzenvorrichtung (1) tendenziell in Richtung der Öffnungen (7) strömt, wenn die Zweikammer-Spritzenvorrichtung (1) mit der Auslassöffnung (5a) erhöht gehalten wird. Ansonsten entspricht das zweite Ausführungsbeispiel dem ersten Ausführungsbeispiel, so dass auf die dort gegebene Beschreibung verwiesen wird.

### Beispiel 3

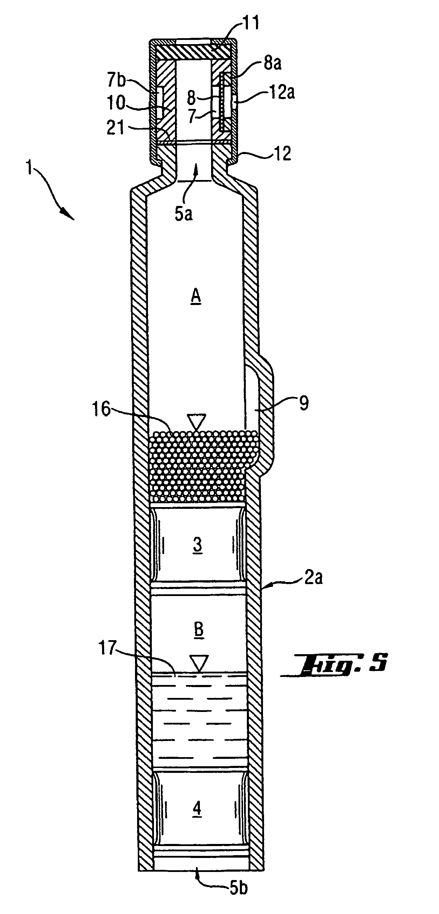

Nachfolgend wird unter Bezugnahme auf Fig. 5 eine Zweikammer-Spritzenvorrichtung (1) gemäß einem dritten Ausführungsbeispiel der Erfindung beschrieben. Wie in der schematischen Schnittansicht von Fig. 5 ersichtlich ist, umfasst die Zweikammer-Spritzenvorrichtung (1) ebenfalls ein Zylinderelement (2) bestehend aus einem Zylinder (2a), zwei Kolben (3) und (4), sowie ein Verschlusselement (6, Ziffer nicht abgebildet). Die fluiddichte, gasdurchlässige Membran (8) befindet sich seitlich im Verschlusselement (6). Das Verschlusselement (6) besteht aus einem Distanzring (10), einer Dichtscheibe (11) und einer Fixierhülse (12), wobei die Bohrung (7) in den Distanzring (10) eingeformt ist und mit einer fluiddichten, gasdurchlässigen Membran (8) verschlossen ist, die von einem Rahmen (8a) fixiert ist. Der Distanzring (10) ist auf den Zylinder (2a) oberhalb der Auslassöffnung (5a) aufgesetzt. Auf den Distanzring (10) ist die Dichtscheibe (11) aufgelegt. Distanzring (10) und Dichtscheibe (11) werden durch die Fixierhülse (12) umschlossen und durch Verbördeln mit dem Zylinder (2a) fest verbunden. Ein weiterer Dichtring (21) ist zwischen Zylinder (2a) und Distanzring (10) eingefügt (abgebildet). Der Dichtring (21) kann weggelassen werden, wenn für den Distanzring (10) ein ausreichend elastisches Material verwendet wird. Zur Erleichterung der Entlüftung ist in den Distanzring (10) auf der Außenseite in Höhe der Bohrung (7) ein umlaufender Entlüftungskanal (7b) eingeformt. Auf gleicher Höhe befindet sich in der Fixierhülse (12) eine Bohrung (12a), die das Entweichen der Luft ermöglicht. Analog zu Ausführungsbeispiel 2 können auf dem Umfang des Distanzrings auch mehrere Bohrungen (7) angeordnet sein, die jeweils mit fluiddichten, gasdurchlässigen Membranen verschlossen sind. Hierdurch kann die verschlossene Zweikammer-Spritzenvorrichtung in vertikaler Lage entlüftet werden, da die mit gasdurchlässiger Membran verschlossene Bohrung (7) in unmittelbarer Nähe der Auslassöffnung angebracht ist und das Gas in der Zweikammer-Spritzenvorrichtung (1) tendenziell in Richtung der Öffnung strömt, wenn die Zweikammer-Spritzenvorrichtung (1) mit der Auslassöffnung (5a) nach oben gehalten wird. Ansonsten entspricht das dritte Ausführungsbeispiel dem ersten Ausführungsbeispiel, so dass auf die dort gegebene Beschreibung verwiesen wird.

### Beispiel 4

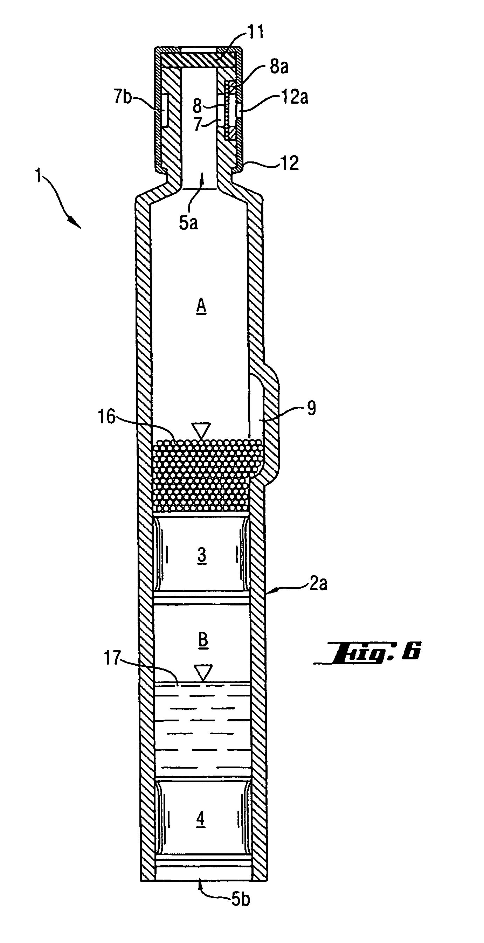

Nachfolgend wird unter Bezugnahme auf Fig. 6 eine Zweikammer-Spritzenvorrichtung (1) gemäß einem weiteren Ausführungsbeispiel der Erfindung beschrieben. Wie in der schematischen Schnittansicht von Fig. 6 ersichtlich ist, umfasst die Zweikammer-Spritzenvorrichtung (1) ein Zylinderelement (2) bestehend aus einem Zylinder (2a), zwei Kolben (3) und (4), sowie ein Verschlusselement (6, Ziffer nicht abgebildet). Die fluiddichte, gasdurchlässige Membran (8) befindet sich seitlich nahe der Auslassöffnung (5a) in der Wand des Zylinders (2a). Das Verschlusselement (6) besteht aus einer Dichtscheibe (11) und einer Fixierhülse (12). Die Bohrung (7) ist im Bereich der Fixierhülse in den Zylinder (2a) eingeformt und wird mit einer fluiddichten, gasdurchlässigen Membran (8) verschlossen. Die Dichtscheibe (11) ist auf die Auslassöffnung (5a) aufgesetzt und wird durch die Fixierhülse (12) umschlossen und durch Verbördeln mit dem Zylinder (2a) fest verbunden. Zur Erleichterung der Entlüftung ist in den Zylinder (2a) auf der Außenseite in Höhe der Bohrung (7) ein umlaufender Entlüftungskanal (7b) eingeformt. Auf gleicher Höhe befindet sich in der Fixierhülse (12) eine Bohrung (7), die das Entweichen der Luft aus dem Zylinderinneren ermöglicht. Analog zu Ausführungsbeispiel 2 können auf dem Umfang des Zylinders (2a) auch mehrere Bohrungen (7) angeordnet sein, die jeweils mit fluiddichten, gasdurchlässigen Membranen verschlossen sind. Hierdurch kann die verschlossene Zweikammer-Spritzenvorrichtung in vertikaler Lage entlüftet werden, da die mit fluiddichter, gasdurchlässiger Membran (8) verschlossene Bohrung (7) in unmittelbarer Nähe der Auslassöffnung (5a) angebracht ist und das Gas in der Zweikammer-Spritzenvorrichtung (1) tendenziell in Richtung der Öffnung strömt, wenn die Zweikammer-Spritzenvorrichtung (1) mit der Auslassöffnung (5a) nach oben gehalten wird. Ansonsten entspricht das Ausführungsbeispiel dem ersten Ausführungsbeispiel, so dass auf die dort gegebene Beschreibung verwiesen wird.

### Beispiel 5

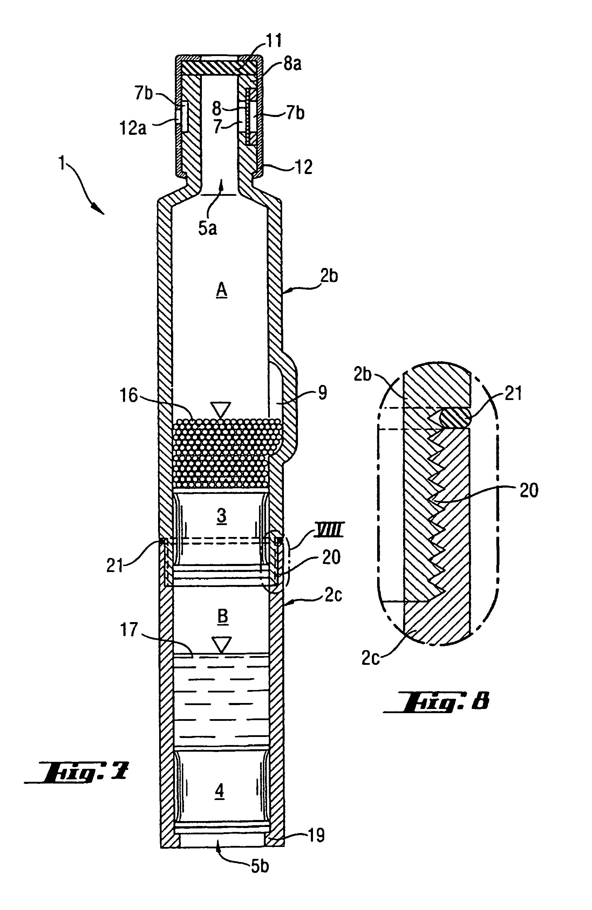

Nachfolgend wird unter Bezugnahme auf den Figuren 7 und 8 eine Zweikammer-Spritzenvorrichtung (1) gemäß einem weiteren Ausführungsbeispiel der Erfindung beschrieben. Wie in der schematischen Schnittansicht von Fig. 7 ersichtlich ist, umfasst die Zweikammer-Spritzenvorrichtung (1) ein zweiteiliges Zylinderelement (2) bestehend aus den Teilzylindern (2b) und (2c), zwei Kolben (3) und (4), sowie ein Verschlusselement (6), in dem die Öffnung (12a) in der Fixierhülse (12) gegenüber der Membran (8) angeordnet ist und Gas über den Entlüftungskanal (7b) abgeleitet wird. Die Teilzylinder sind mittels Schraubverbindung miteinander verbunden (Fig. 8). Zur Abdichtung wird ein Dichtring (21) verwendet, der zwischen den Teilzylindern eingelegt ist. Das proximale Ende des Teilzylinders (2b) schließt mindestens mit dem proximalen Ende des Kolbens (3) ab. Teilzylinder (2b) enthält oberhalb des distalen Endes des Kolbens (3) im Bereich der Kammer A einen Bypass (9). Ansonsten entspricht dieses Ausführungsbeispiel dem Ausführungsbeispiel 4, so dass auf die dort gegebene Beschreibung verwiesen wird.

### Beispiel 6

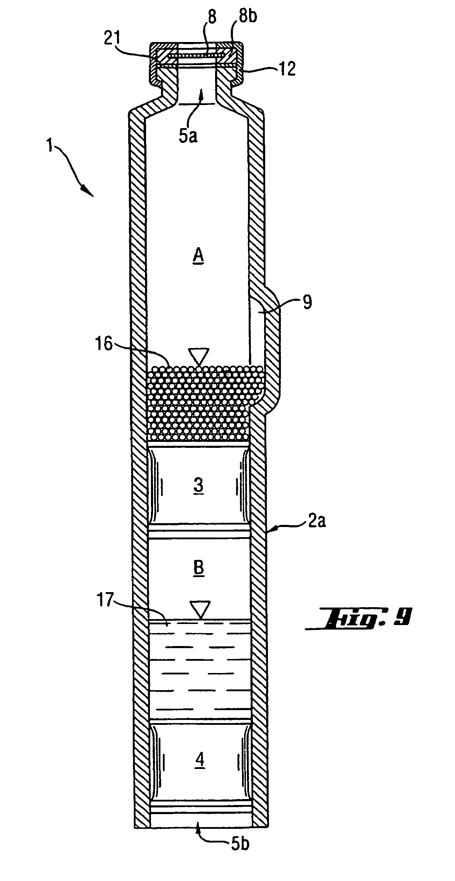

Nachfolgend wird unter Bezugnahme auf Fig. 9 eine Zweikammer-Spritzenvorrichtung (1) gemäß einem weiteren Ausführungsbeispiel der Erfindung beschrieben. Wie in der schematischen Schnittansicht von Fig. 9 ersichtlich ist, umfasst die Zweikammer-Spritzenvorrichtung (1) ein Zylinderelement (2) bestehend aus einem Zylinder (2a), zwei Kolben (3) und (4), sowie ein Verschlusselement (6, Ziffer nicht abgebildet). Die fluiddichte, gasdurchlässige Membran (8) ist axial im Verschlusselement (6) angeordnet. Das Verschlusselement (6) besteht aus einer fluiddichten, gasdurchlässigen Membran (8) und einer Fixierhülse (12). Die fluiddichte, gasdurchlässige Membran (8) ist auf die Auslassöffnung (5a) aufgesetzt. Die fluiddichte, gasdurchlässige Membran (8) wird in einer Blende (8b) gehaltert und die Blende mit der Membran wird durch die Fixierhülse (12) umschlossen und durch Verbördeln mit dem Zylinder (2a) fest verbunden. Im vorliegenden Beispiel ist der Übergang zwischen Blende (8b) und Zylinder (2a) durch einen Dichtring (21) abgedichtet. Alternativ kann der Dichtring auch weggelassen werden, wenn für die Blende ein elastisches Material verwendet wird, beispielsweise ein für die Kolben (3) und (4) oder die Dichtscheibe (11) beschriebenes Material, *vide supra.*

Durch die Positionierung der Membran kann die verschlossene Zweikammer-Spritzenvorrichtung in vertikaler Lage vollständig entlüftet werden, da die mit der fluiddichten, gasdurchlässigen Membran (8) verschlossene Öffnung gleichzeitig die Auslassöffnung darstellt und das Gas in der Zweikammer-Spritzenvorrichtung (1) tendenziell in Richtung der Öffnung strömt, wenn die Zweikammer-Spritzenvorrichtung (1) mit der Auslassöffnung (5a) nach oben gehalten wird. Ansonsten entspricht das vierte Ausführungsbeispiel dem ersten Ausführungsbeispiel, so dass auf die dort gegebene Beschreibung verwiesen wird.

### Beispiel 7

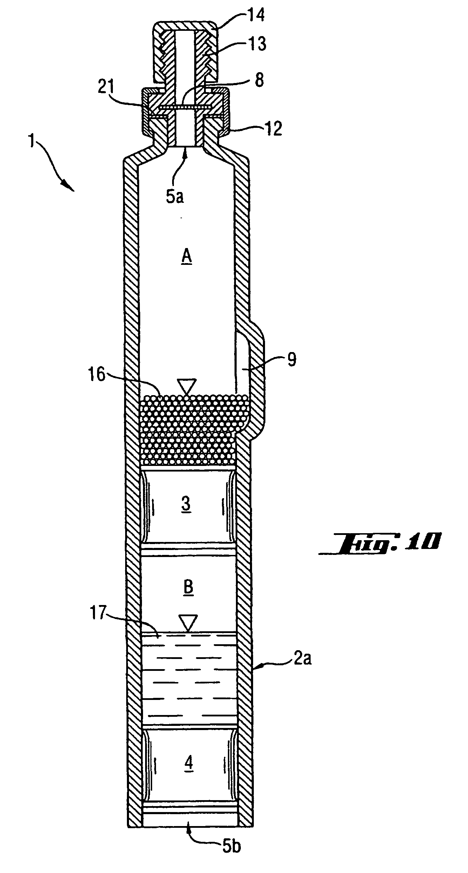

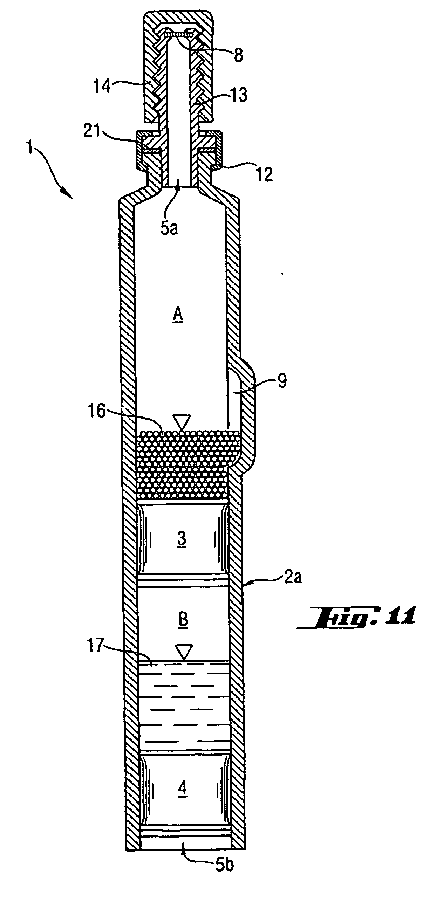

Nachfolgend wird unter Bezugnahme auf die Figuren 10 und 11 eine Zweikammer-Spritzenvorrichtung (1) gemäß einem weiteren Ausführungsbeispiel der Erfindung beschrieben. Wie in der schematischen Schnittansicht von Fig. 10 und 11 ersichtlich ist, umfasst die Zweikammer-Spritzenvorrichtung (1) ein Zylinderelement (2) bestehend aus einem Zylinder (2a), zwei Kolben (3) und (4), sowie ein Verschlusselement (6, Ziffer nicht abgebildet). Das Verschlusselement (6) besteht aus einer Fixierhülse (12), einem Gewindeformteil (13), in dem eine fluiddichte, gasdurchlässige Membran (8) fixiert ist, und optional einer Schutzkappe (14). Das Gewindeformteil (13) mit integrierter fluiddichter, gasdurchlässiger Membran (8) wird auf die Auslassöffnung (5a) aufgesetzt. Mit der Fixierhülse (12) wird das Gewindeformteil (13) durch Verbördeln mit dem Zylinder (2a) fest verbunden, wobei der Übergang zwischen Gewindeformteil (13) und Zylinder (2a) zusätzlich durch einen Dichtring (21) abgedichtet ist. Das Gewindeformteil (13) ist vorzugsweise durch eine abnehmbare Schutzkappe (14) verschlossen und erlaubt das Aufschrauben einer Injektionsnadel nach Abnehmen der Schutzkappe (14). Hierdurch kann die verschlossene Zweikammer-Spritzenvorrichtung in vertikaler Lage vollständig entlüftet werden, da die mit der fluiddichten, gasdurchlässigen Membran (8) verschlossene Öffnung gleichzeitig die Auslassöffnung darstellt und das Gas in der Zweikammer-Spritzenvorrichtung (1) tendenziell in Richtung der Öffnung strömt, wenn die Zweikammer-Spritzenvorrichtung (1) mit der Auslassöffnung (5a) nach oben gehalten wird.

In Fig. 10 befindet sich die Membran (8) in der Mitte des Gewindeformteils (13) an einer Stelle, an der Bauteil (13) seinen größten Außendurchmesser aufweist. Auf diese Weise kann beispielsweise die Membran (8) in das Gewindeformteil (13) eingegossen werden. Beispielsweise kann das integrierte Gewindeformteil (13) gefertigt werden, indem die Membran (8) auf den distalen Flansch eines proximalen Teils des Gewindeformteils gelegt wird, und das proximale Gegenstück des Gewindeformteils enthaltend das Gewinde und einen proximalen Flansch auf die Membran und den distalen Flansch aufgelegt und mit diesen verschmolzen wird (nicht gezeigt).

Fig. 11 zeigt die Position der Membran (8) am äußeren Ende des Gewindeformteils (13).

### Beispiel 8

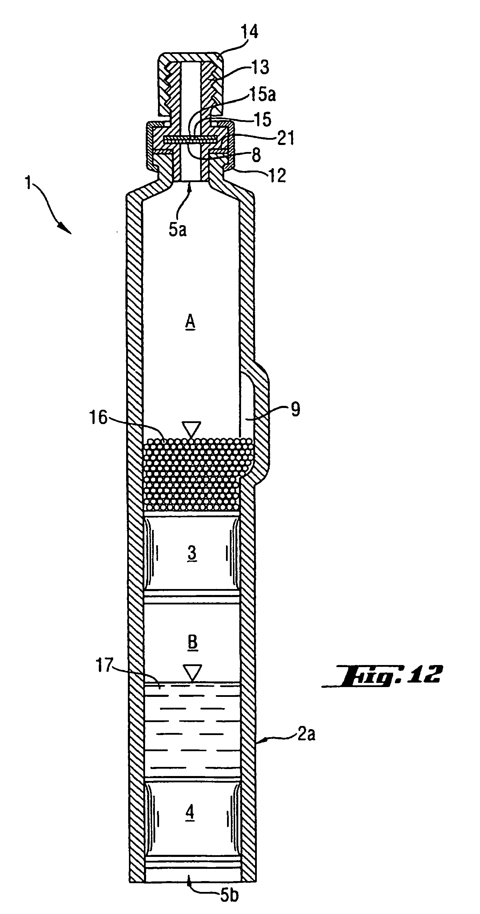

Nachfolgend wird unter Bezugnahme auf die Figuren 12 bis 15 eine Zweikammer-Spritzenvorrichtung (1) beschrieben, die der in Beispiel 7 beschriebenen entspricht, wobei aber zusätzlich eine Ventilmembran (15) auf der nach außen gerichteten Seite der Membran (8) angeordnet ist. Die Ventilmembran (15) schützt die darunter liegende fluiddichte, gasdurchlässige Membran (8) und begrenzt die Gasdiffusion aus der Lagerumgebung in das Primärpackmittel.

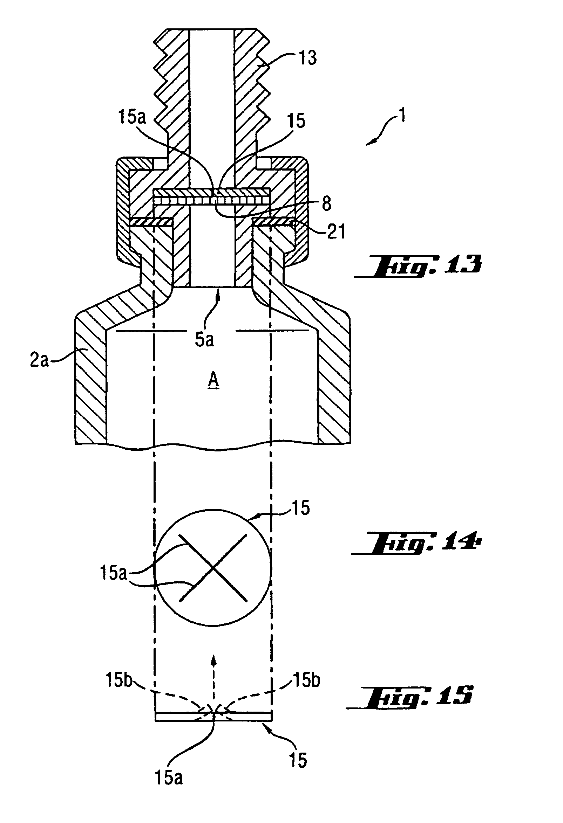

Fig. 13 zeigt einen vergrößerten Ausschnitt des Kopfbereiches der Zweikammer-Spritzenvorrichtung dieses Beispiels. Fig. 14 zeigt eine Aufsicht auf die Ventilmembran (15). Die Ventilfunktion wird durch kreuzförmig gestaltete Stanzlinien (15a) in der Membran hergestellt. Ein innerhalb der Zweikammer-Spritzenvorrichtung (1) entstehender Überdruck entweicht durch die gasdurchlässige Membran (8) durch Öffnung der Ventilmembran (15) entlang der Stanzlinien (15a). Die geöffnete Position der Ventilmembran bei Gasdurchtritt ist in der Seitenansicht in Fig. 15 durch die Position der geöffneten Ventilmembran (15b) angedeutet.

### Beispiel 9

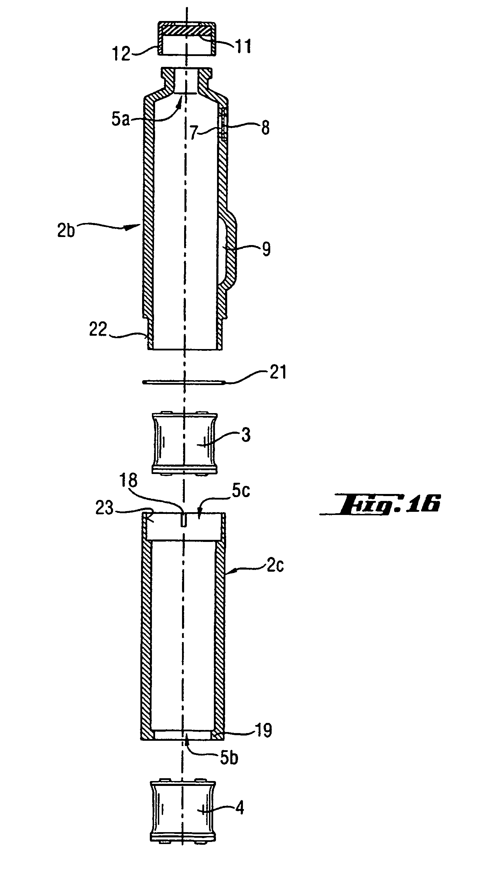

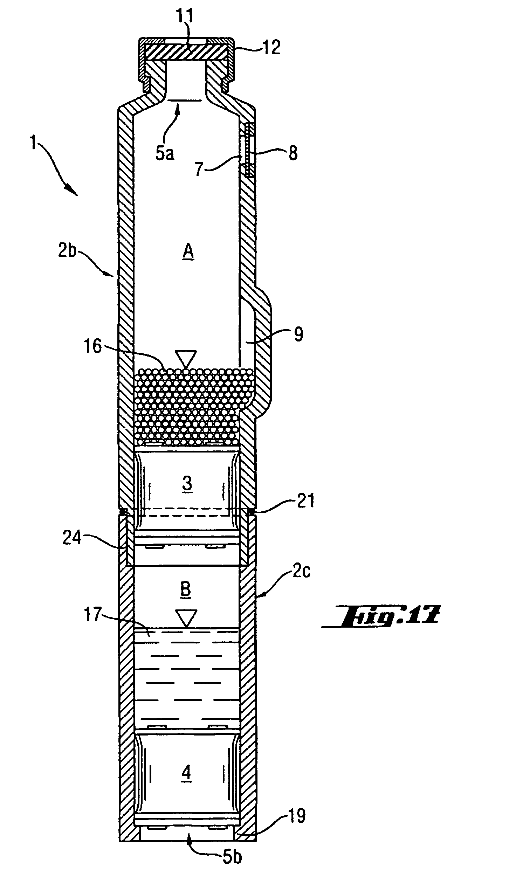

Nachfolgend wird unter Bezugnahme auf die Figuren 16 und 17 eine Zweikammer-Spritzenvorrichtung (1) beschrieben, in der das Zylinderelement (2) zweiteilig ist und aus einem distalen Teilzylinder (2b) und einem proximalen Teilzylinder (2c) besteht, und die ferner zwei Kolben (3) und (4) und ein Verschlusselement (6) umfasst. Die Teilzylinder sind durch eine Steckverbindung an der Verbindungsstelle (24) fest miteinander verbunden und durch einen Dichtring (21) zusätzlich abgedichtet. Die fluiddichte, gasdurchlässige Membran (8) befindet sich seitlich unterhalb des Schulterbereichs in der Nähe des distalen Endes des Teilzylinders (2b).

Der Bypass (9) befindet sich in dieser Ausführungsform in der Wand des distalen Teilzylinders und ist so dimensioniert, dass die Länge des Bypasses größer ist als die Länge des Kolbens (3).

Der proximale Teilzylinder (2c) weist im Bereich der Öffnung (5b) einen Anschlag (19) auf, der verhindern soll, dass beim Zusammenfügen der Teilzylinder (2b) und (2c) der Kolben (4) durch den sich in der Kammer B aufbauenden Druck herausgedrückt wird.

Im Bereich der Erweiterung (23) in Teilzylinders (2c), in die der Absatz (22) in Teilzylinder (2b) eingeführt wird, befindet sich eine axiale Nut, die eine Länge von 50 bis 90 % der Länge der Erweiterung (23) hat und über die der Druck, der sich beim Zusammenfügen der beiden Teilzylinder aufbaut, zusätzlich vermindert wird.

Für die Beschreibung der Anwendung wird auf Beispiel 1 verwiesen. Fig. 17 zeigt das Ausführungsbeispiel im montierten Zustand mit Füllung der Kammern A (hier: feste Komponente) und B (flüssige Komponente).

### Beispiel 10

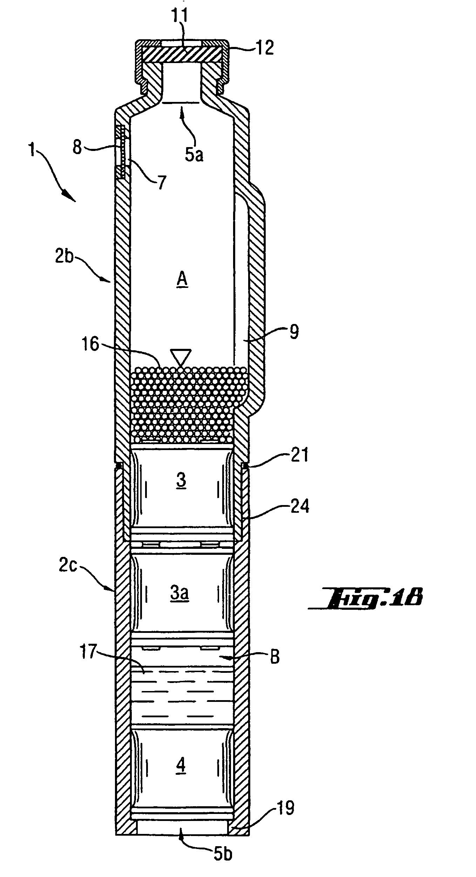

Nachfolgend wird unter Bezugnahme auf Fig. 18 eine Zweikammer-Spritzenvorrichtung (1) beschrieben, in der das Zylinderelement (2) zweiteilig ist und aus einem distalen Teilzylinder (2b) und einem proximalen Teilzylinder (2c) besteht, und die ferner drei Kolben (3), (3a) und (4) und ein Verschlusselement (6) umfasst. Die Teilzylinder sind durch eine Steckverbindung (24) fest miteinander verbunden und durch einen Dichtring (21) zusätzlich abgedichtet. Die fluiddichte, gasdurchlässige Membran (8) befindet sich seitlich unterhalb des Schulterbereichs in der Nähe des distalen Endes des Teilzylinders (2b). Vorzugsweise ist die Membran (8) an der dem Bypass (9) gegenüberliegenden Seite positioniert. Das Ausführungsbeispiel entspricht ansonsten der in Beispiel 1 genannten Zweikammer-Spritzenvorrichtung. Fig. 18 zeigt das Ausführungsbeispiel im montierten Zustand mit Füllung der Kammern A (hier: feste Komponente) und B.

Der Bypass (9) befindet sich in dieser Ausführungsform in der Wand des distalen Teilzylinders und ist so dimensioniert, dass die Länge des Bypasses größer ist als die Summe der Längen der Kolben (3) und (3a).

Der proximale Teilzylinder (2c) weist im Bereich der Öffnung (5b) einen Anschlag (19) auf, der verhindern soll, dass beim Einfügen des Kolbens (3a) der Kolben (4) durch den sich in der Kammer B aufbauenden Druck herausgedrückt wird.

Fig. 18 zeigt das Ausführungsbeispiel im montierten Zustand mit Füllung der Kammern A (hier: feste Komponente) und B (flüssige Komponente).

Nachfolgend wird die Montage und das Befüllen der erfindungsgemäßen Zweikammer-Spritzenvorrichtung (1) beschrieben:

Für die Montage des distalen Teils der Zweikammer-Spritzenvorrichtung wird zunächst die Dichtscheibe (11) mittels der Fixierhülse (12) am distalen Ende eines Teilzylinders (2b), in den eine gasdurchlässige, fluiddichte Membran (8) eingearbeitet ist, fest verbunden. Dann wird durch die proximale Öffnung des Teilzylinders (2b) eine feste (abgebildet) oder alternativ flüssige Komponente in Kammer A eingefüllt, und die proximale Öffnung des Teilzylinders wird mit Kolben (3) verschlossen.

Für die Montage des proximalen Teils der Zweikammer-Spritzenvorrichtung wird zunächst der Kolben (4) in den Teilzylinder (2c) eingeführt und an dessen proximalen Ende positioniert. Dann wird die Kammer B mit einem flüssigen Medium gefüllt. Anschließend wird der Kolben (3a) in die distale Öffnung des Teilzylinders eingeführt und Kammer B damit verschlossen.

Vor der Anwendung der Zweikammer-Spritzenvorrichtung wird das proximale Ende des distalen Teilzylinders (2b) mit dem distalen Ende des proximalen Teilzylinders (2c) fest miteinander verbunden, wobei die Verbindung zusätzlich mit einem Dichtring (21) abgedichtet werden kann.

Die beiden Teilzylinder (2b) und (2c) können voneinander getrennt gefüllt und bis zur Montage separat gelagert werden. Dies hat den Vorteil, dass Kontaminationen von Kammer B mit der festen oder flüssigen Komponente in Kammer A vermieden wird. Ferner lassen sich die logistischen Abläufe in einer großtechnischen Fertigung leichter planen und durchführen. Ferner kann Teilzylinder (2c) über eine Öffnung befüllt werden, die die Größe des gesamten inneren Durchmesser des Teilzylinders (2c) hat. Dadurch wird es ermöglicht, insbesondere feste Komponenten auf vereinfachte Art und Weise abzufüllen, ohne dass ein Befüllen durch die distale Auslassöffnung (5a) und anschließendes Lyophilisieren nötig wäre.

Für die Beschreibung der Anwendung wird auf Beispiel 1 verwiesen.

### Beispiel 11

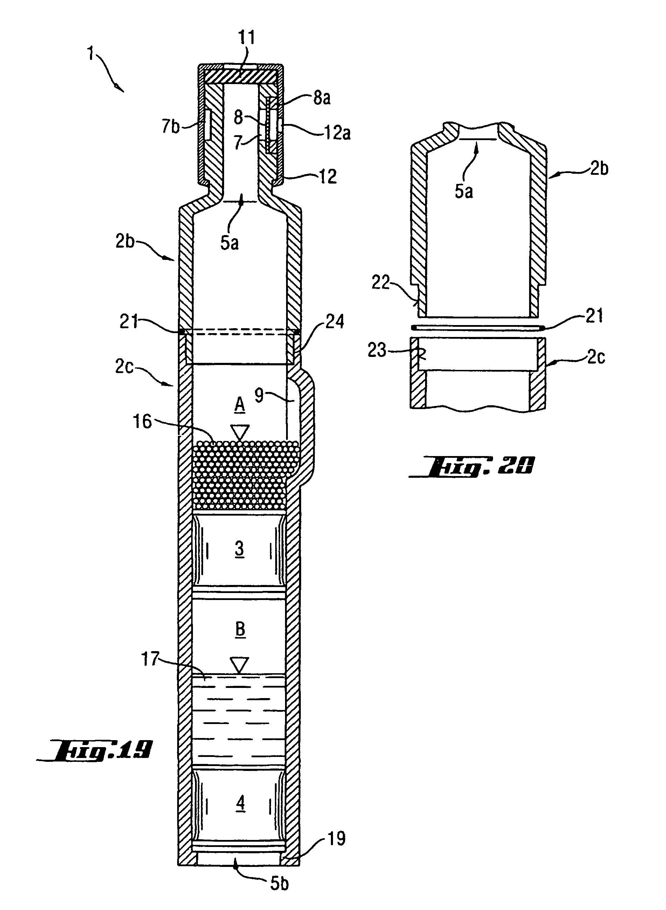

Nachfolgend wird unter Bezugnahme auf die Figuren 19 und 20 eine weitere Zweikammer-Spritzenvorrichtung (1) beschrieben, die ein zweiteiliges Zylinderelement (2) bestehend aus einem distalen Teilzylinder (2b) und einem proximalen Teilzylinder (2c), zwei Kolben (3) und (4), sowie ein Verschlusselement (6, Ziffer nicht abgebildet) umfasst.

Die fluiddichte, gasdurchlässige Membran (8) befindet sich seitlich nahe der Auslassöffnung (5a) in der Wand des Zylinders (2a). Das Verschlusselement (6) besteht aus einer Dichtscheibe (11) und einer Fixierhülse (12). Die Bohrung (7) ist im Bereich der Fixierhülse in Teilzylinder (2b) eingeformt und wird mit einer fluiddichten, gasdurchlässigen Membran (8) verschlossen. Die Dichtscheibe (11) ist auf die Auslassöffnung (5a) aufgesetzt und wird durch die Fixierhülse (12) umschlossen und durch Verbördeln mit dem Teilzylinder (2a) fest verbunden. Zur Erleichterung der Entlüftung ist in (2b) auf der Außenseite in Höhe der Bohrung (7) ein umlaufender Entlüftungskanal (7b) eingeformt. Auf gleicher Höhe befindet sich in der Fixierhülse (12) eine Bohrung (7), die das Entweichen der Luft aus dem Zylinderinneren ermöglicht. Bohrung (7) und Membran (8) können dabei übereinander zu liegen kommen, bevorzugt kommen Bohrung (7) und Membran (8) aber nicht zur Deckung, damit die Membran (8) durch die Fixierhülse einen weiteren mechanischen Schutz erhält. Analog zu Ausführungsbeispiel 2 können auf dem Umfang des Teilzylinders (2b) auch mehrere Bohrungen (7) angeordnet sein, die jeweils mit fluiddichten, gasdurchlässigen Membranen verschlossen sind.

Fig. 19 zeigt das Ausführungsbeispiel im montierten Zustand mit Füllung der Kammern A (hier: feste Komponente) und B. Fig. 20 zeigt eine Detailvergrößerung der Verbindungsstelle der beiden Teilzylinder (2b) und (2c).

Der Bypass (9) befindet sich in dieser Ausführungsform in der Wand des proximalen Teilzylinders (2c) und ist so dimensioniert, dass die Länge des Bypasses größer ist als Länge des Kolbens (3).

Der proximale Teilzylinder (2c) weist im Bereich der Öffnung (5b) einen Anschlag (19) auf, der verhindern soll, dass beim Einfügen des Kolbens (3) der Kolben (4) durch den sich in der Kammer B aufbauenden Druck herausgedrückt wird.

Nachfolgend wird die Montage und das Befüllen der erfindungsgemäßen Zweikammer-Spritzenvorrichtung (1) beschrieben:

Für die Montage des distalen Teils der Zweikammer-Spritzenvorrichtung (1) wird zunächst eine Dichtscheibe (11) mittels einer Fixierhülse (12) am distalen Ende eines Teilzylinders (2b), in den eine gasdurchlässige, fluiddichte Membran (8) eingearbeitet ist, fest verbunden.

Für die Montage des proximalen Teils der Zweikammer-Spritzenvorrichtung wird zunächst der Kolben (4) in den Teilzylinder (2c) eingeführt und an dessen proximalen Ende positioniert. Dann wird die Kammer B mit einem flüssigen Medium gefüllt. Anschließend wird der Kolben (3) durch die distale Öffnung des Teilzylinders eingeführt und Kammer B damit verschlossen. Dann wird durch die distale Öffnung des Teilzylinders (2c) eine feste (abgebildet) oder flüssige Komponente in Kammer A eingefüllt, und die distale Öffnung durch Aufsetzen des vormontierten distalen Teils der Zweikammer-Spritzenvorrichtung verschlossen, wobei die Verbindung zusätzlich mit einem Dichtring (21) abgedichtet werden kann. Beim Aufsetzen des Teilzylinders (2c) entsteht kein Überdruck in Kammer A, da die eingeschlossene Luft durch die Membran (8) entweichen kann.

Teilzylinder (2c) kann über eine Öffnung, die die Größe des gesamten inneren Durchmesser des Teilzylinders (2c) hat, befüllt werden. Dadurch wird es ermöglicht, insbesondere feste Komponenten auf vereinfachte Art und Weise abzufüllen, ohne dass ein Befüllen durch die distale Auslassöffnung (5a) und anschließendes Lyophilisieren nötig wäre.

Für die Beschreibung der Anwendung wird auf Beispiel 1 verwiesen.

**Bezugszeichen:**

| | |
|---|---|
| A | Kammer zwischen Verschlusselement (6) und dem Kolben (3) und begrenzt durch die Wand des Zylinderelements (2) |
| B | Kammer zwischen Kolben (3) oder (3a) und Kolben (4) und begrenzt durch die Wand des Zylinderelements (2) |
| 1 | Zweikammer-Spritzenvorrichtung |
| 2 | Zylinderelement |
| 2a | Zylinder |
| 2b | erster, distaler Teilzylinder |
| 2c | zweiter, proximaler Teilzylinder |
| 3 | distaler Kolben |
| 3a | zusätzlicher Kolben als Verschluss des proximalen Teilzylinders (2c) |
| 4 | proximaler Kolben |
| 5a | distale Auslassöffnung in Zylinder (2a) |
| 5b | proximale Öffnung im Zylinder (2a) |
| 5c | proximale Öffnung im ersten, distalen Teilzylinder (2b) |
| 5d | distale Öffnung im zweiten, proximalen Teilzylinder (2c) |
| 6 | Verschlusselement der Auslassöffnung (5a) |
| 7 | Bohrung |
| 7a | Schulter |
| 7b | Entlüftungskanal |
| 8 | fluiddichte, gasdurchlässige Membran |
| 8a | Rahmen zur Fixierung der Membran (8) |
| 8b | Blende |
| 9 | Überleitungskanal (Bypass) |
| 10 | Distanzring |
| 11 | Dichtscheibe |
| 12 | Fixierhülse |
| 12a | Bohrung in der Fixierhülse (12) |
| 13 | Gewindeformteil |
| 14 | Schutzkappe |
| 15 | Ventilmembran |
| 15a | Stanzschnitt bzw. Stanzöffnung in der Ventilmembran (15) |
| 15b | geöffnete Position der Ventilmembran (15) bei Gasdurchtritt |
| 16 | feste oder flüssige Komponente in Kammer A |
| 17 | flüssige Komponente in Kammer B |
| 18 | axiale Nut |
| 19 | Anschlag |
| 20 | Schraubverbindung |
| 21 | Dichtring |
| 22 | Absatz am proximalen Ende des Teilzylinders (2b) |
| 23 | Erweiterung am distalen Ende des Teilzylinders (2c) |
| 24 | Verbindungszone der Teilzylinder (2b) // (2c) |

## Patentansprüche

1. Zweikammer-Spritzenvorrichtung umfassend
a) ein Zylinderelement (2) mit einer distalen Auslassöffnung (5a), einer proximalen Öffnung (5b) und einem oder mehreren Überleitungskanälen (9),
b) ein Verschlusselement (6) der distalen Auslassöffnung (5a) umfassend eine Dichtungscheibe (11) und eine Fixierhülse (12), und
c) einem distalen Kolben (3) und einem proximalen Kolben (4), welche in das Zylinderelement (2) einführbar sind,
wobei zwischen dem Verschlusselement (6) und dem distalen Kolben (3) innerhalb des Zylinderelements (2) eine Kammer A gebildet wird,
Wobei in der Wandung des Zylinderelementes (2) oder in dem Verschlusselement (6) eine oder mehrere fluiddichte, gasdurchlässige Membranen (8) angeordnet sind, **dadurch gekennzeichnet, dass** die Spritzenvorrichtung als Karpule ausgebildet ist, die in ein dafür vorgesehenes, mehrfach verwendbares Injektor-System mit einem Antriebsmechanismus zum Bewegen des Kolbens (4) in distaler Richtung einsetzbar ist.

2. Zweikammer-Spritzenvorrichtung nach Anspruch 1, **dadurch gekennzeichnet, dass** das Zylinderelement (2) einteilig ausgeformt ist und aus dem Zylinder (2a) besteht.

3. Zweikammer-Spritzenvorrichtung nach Anspruch 1, **dadurch gekennzeichnet, dass** das Zylinderelement (2) zweiteilig ausgeformt ist und aus dem distalen Teilzylinder (2b) und dem proximalen Teilzylinder (2c) besteht.

4. Zweikammer-Spritzenvorrichtung nach Anspruch 3, **dadurch gekennzeichnet, dass** zwischen Kolben (3) und (4) ein weiterer Kolben (3a) am distalen Ende des proximalen Teilzylinders (2c) angeordnet ist.

5. Zweikammer-Spritzenvorrichtung nach einem der Ansprüche 1 bis 4, wobei das Verschlusselement (6) ein Element zum Befestigen einer Nadel enthält.

6. Zweikammer-Spritzenvorrichtung nach einem der Ansprüche 1 bis 5, wobei das Verschlusselement (6) einen Distanzring (10) enthält, der zwischen Dichtscheibe (11) und Zylinderelement (2) positioniert ist, und optional einen Dichtring (21) zwischen Distanzring (10) und Zylinderelement (2) enthält.

7. Zweikammer-Spritzenvorrichtung nach einem der Ansprüche 1 bis 6, wobei die Zweikammer-Spritzenvorrichtung eine Schutzkappe (14) zum Aufsatz auf das distale Ende des Zylinderelements (2) und/oder das distale Ende des Verschlusselementes (6) enthält.

8. Zweikammer-Spritzenvorrichtung nach einem der Ansprüche 1 bis 7, **dadurch gekennzeichnet, dass** die fluiddichte, gasdurchlässige Membran (8) in der Wandung des Zylinderelementes (2) am distalen Ende des Zylinderelementes (2) im Bereich der Kammer A angeordnet ist.

9. Zweikammer-Spritzenvorrichtung nach einem der Ansprüche 1 bis 7, **dadurch gekennzeichnet, dass** die fluiddichte, gasdurchlässige Membran (8) seitlich im Verschlusselement (6) angeordnet ist.

10. Zweikammer-Spritzenvorrichtung nach einem der Ansprüche 1 bis 7 **dadurch gekennzeichnet, dass** das Verschlusselement (6) eine Dichtscheibe (11) und zusätzlich die fluiddichte, gasdurchlässige Membran (8) enthält.

11. Zweikammer-Spritzenvorrichtung nach einem der Ansprüche 1 bis 7, **dadurch gekennzeichnet, dass** die Dichtscheibe (11) die fluiddichte, gasdurchlässige Membran (8) darstellt.

12. Zweikammer-Spritzenvorrichtung nach einem der Ansprüche 1 bis 11, **dadurch gekennzeichnet, dass** über der fluiddichten, gasdurchlässigen Membran (8) ein Ventilelement (15) angeordnet ist.

13. Zweikammer-Spritzenvorrichtung nach einem der Ansprüche 1 bis 12, **dadurch gekennzeichnet, dass** die fluiddichte, gasdurchlässige Membran (8) eine Sterilfiltermembran ist.

14. Zweikammer-Spritzenvorrichtung nach Anspruch 13, wobei die Sterilfiltermembran eine Porengröße von kleiner gleich 0,2 µm hat.

15. Zweikammer-Spritzenvorrichtung nach einem der Ansprüche 1 bis 14, **dadurch gekennzeichnet, dass** die distale Kammer A eine feste Komponente enthält und die proximale Kammer B eine flüssige Komponente.

16. Zweikammer-Spritzenvorrichtung nach einem der Ansprüche 1 bis 14, **dadurch gekennzeichnet, dass** beide Kammern A und B eine flüssige Komponente enthalten.

17. Anordnung mit einer Zweikammer-Spritzenvorrichtung gemäß einem der Ansprüche 1 bis 16 und einer Umverpackung für die Zweikammer-Spritzenvorrichtung, **dadurch gekennzeichnet, dass** die Umverpackung aus einem gasdichten Folienmaterial hergestellt ist.

18. Anordnung gemäß Anspruch 17, **dadurch gekennzeichnet, dass** das Umverpackungsmaterial Aluminiumfolie, kunststofflaminierte Aluminiumfolie oder aluminiumbeschichtete Kunststofffolie ist.

19. Applikationsvorrichtung enthaltend eine Zweikammer-Spritzenvorrichtung gemäß einem der Ansprüche 1 bis 16, eine Nadel zum Aufsetzen auf das distale Ende des Zylinderelementes (2) und optional einen oder mehrere Desinfektions-Tupfer.

## Claims

1. A two-chamber syringe device comprising
a) a cylinder element (2) with a distal outlet opening (5a), a proximal opening (5b) and one or more transfer channels (9),
b) a closure element (6) of the distal outlet opening (5a) comprising a sealing disk (11) and a fixing sleeve (12), and
c) a distal plunger (3) and a proximal plunger (4), which can be introduced into the cylinder element (2),
a chamber A being formed between the closure element (6) and the distal plunger (3) inside the cylinder element (2),
one or more fluid-tight, gas-permeable membranes (8) being arranged in the wall of the cylinder element (2) or in the closure element (6), wherein the syringe device is formed as a carpule which can be used in a multiple-use injector system provided therefor and having a drive mechanism for moving the plunger (4) in the distal direction.

2. The two-chamber syringe device as claimed in claim 1, wherein the cylinder element (2) is formed as one part and comprises the cylinder (2a).

3. The two-chamber syringe device as claimed in claim 1, wherein the cylinder element (2) is formed as two parts and comprises the distal part-cylinder (2b) and the proximal part-cylinder (2c).

4. The two-chamber syringe device as claimed in claim 3, wherein, between plungers (3) and (4), a further plunger (3a) is arranged at the distal end of the proximal part-cylinder (2c).

5. The two-chamber syringe device as claimed in one of claims 1 to 4, the closure element (6) including an element for fastening a needle.

6. The two-chamber syringe device as claimed in one of claims 1 to 5, the closure element (6) including a spacer ring (10), which is positioned between the sealing disk (11) and the cylinder element (2), and optionally includes a sealing ring (21) between the spacer ring (10) and the cylinder element (2).

7. The two-chamber syringe device as claimed in one of claims 1 to 6, the two-chamber syringe device including a protective cap (14) for fitting onto the distal end of the cylinder element (2) and/or the distal end of the closure element (6).

8. The two-chamber syringe device as claimed in one of claims 1 to 7, wherein the fluid-tight, gas-permeable membrane (8) is arranged in the wall of the cylinder element (2) at the distal end of the cylinder element (2) in the region of the chamber A.

9. The two-chamber syringe device as claimed in one of claims 1 to 7, wherein the fluid-tight, gas-permeable membrane (8) is arranged laterally in the closure element (6).

10. The two-chamber syringe device as claimed in one of claims 1 to 7, wherein the closure element (6) includes a sealing disk (11) and additionally the fluid-tight, gas-permeable membrane (8).

11. The two-chamber syringe device as claimed in one of claims 1 to 7, wherein the sealing disk (11) represents the fluid-tight, gas-permeable membrane (8).

12. The two-chamber syringe device as claimed in one of claims 1 to 11, wherein a valve element (15) is arranged over the fluid-tight, gas-permeable membrane (8).

13. The two-chamber syringe device as claimed in one of claims 1 to 12, wherein the fluid-tight, gas-permeable membrane (8) is a sterile filter membrane.

14. The two-chamber syringe device as claimed in claim 13, the sterile filter membrane having a pore size of less than or equal to 0.2 µm.

15. The two-chamber syringe device as claimed in one of claims 1 to 14, wherein the distal chamber A contains a solid component and the proximal chamber B contains a liquid component.

16. The two-chamber syringe device as claimed in one of claims 1 to 14, wherein both chambers A and B contain a liquid component.

17. An arrangement having a two-chamber syringe device as claimed in one of claims 1 to 16 and an outer packaging for the two-chamber syringe device, wherein the outer packaging is produced from a gas-tight sheet material.

18. The arrangement as claimed in claim 17, wherein the outer packaging material is aluminum foil, plastic-laminated aluminum foil or aluminum-coated plastic film.

19. An applicator including a two-chamber syringe device as claimed in one of claims 1 to 16, a needle for attaching to the distal end of the cylinder element (2) and optionally one or more disinfection pads.

## Revendications

1. Système de seringue à deux chambres, comportant
a) un élément de cylindre (2) avec une ouverture de sortie distale (5a), une ouverture proximale (5b) et un ou plusieurs canaux de transfert (9),
b) un élément de fermeture (6) de l'extrémité de sortie distale (5a) comprenant un disque d'étanchéité (11) et une douille de fixation (12), et
c) un piston distal (3) et un piston proximal (4), qui peuvent être introduits dans l'élément de cylindre (2),
dans lequel une chambre A est formée à l'intérieur de l'élément de cylindre (2) entre l'élément de fermeture (6) et le piston distal (3), dans lequel une ou plusieurs membranes (8) perméables aux gaz et étanches aux fluides sont disposées dans la paroi de l'élément de cylindre (2) ou dans l'élément de fermeture (6),
**caractérisé en ce que** le système de seringue est réalisé sous forme de carpule, qui peut être insérée dans un système d'injecteur utilisable plusieurs fois prévu à cet effet, avec un mécanisme d'entraînement pour le déplacement du piston (4) dans la direction distale

2. Système de seringue à deux chambres selon la revendication 1, **caractérisé en ce que** l'élément de cylindre (2) est formé en une seule pièce et se compose du cylindre (2a).

3. Système de seringue à deux chambres selon la revendication 1, **caractérisé en ce que** l'élément de cylindre (2) est formé en deux pièces et se compose du cylindre partiel distal (2b) et du cylindre partiel proximal (2c).

4. Système de seringue à deux chambres selon la revendication 3, **caractérisé en ce qu'**un autre piston (3a) est disposé entre les pistons (3) et (4) à l'extrémité distale du cylindre partiel proximal (2c).

5. Système de seringue à deux chambres selon l'une quelconque des revendications 1 à 4, dans lequel l'élément de fermeture (6) contient un élément pour la fixation d'une aiguille.

6. Système de seringue à deux chambres selon l'une quelconque des revendications 1 à 5, dans lequel l'élément de fermeture (6) comprend une bague d'écartement (10), qui est positionnée entre le disque d'étanchéité (11) et l'élément de cylindre (2), et comprend en option une bague d'étanchéité (21) entre la bague d'écartement (10) et l'élément de cylindre (2).

7. Système de seringue à deux chambres selon l'une quelconque des revendications 1 à 6, dans lequel le système de seringue à deux chambres comprend un capuchon de protection (14) à poser sur l'extrémité distale de l'élément de cylindre (2) et/ou sur l'extrémité distale de l'élément de fermeture (6).

8. Système de seringue à deux chambres selon l'une quelconque des revendications 1 à 7, **caractérisé en ce que** la membrane perméable aux gaz et étanche aux fluides (8) est disposée dans la paroi de l'élément de cylindre (2), à l'extrémité distale de l'élément de cylindre (2) dans la région de la chambre A.

9. Système de seringue à deux chambres selon l'une quelconque des revendications 1 à 7, **caractérisé en ce que** la membrane perméable aux gaz et étanche aux fluides (8) est disposée latéralement dans l'élément de fermeture (6).

10. Système de seringue à deux chambres selon l'une quelconque des revendications 1 à 7, **caractérisé en ce que** l'élément de fermeture (6) comprend un disque d'étanchéité (11) et en plus la membrane perméable aux gaz et étanche aux fluides (8).

11. Système de seringue à deux chambres selon l'une quelconque des revendications 1 à 7, **caractérisé en ce que** le disque d'étanchéité (11) représente la membrane perméable aux gaz et étanche aux fluides (8).

12. Système de seringue à deux chambres selon l'une quelconque des revendications 1 à 11, **caractérisé en ce qu'**un élément de soupape (15) est disposé au-dessus de la membrane perméable aux gaz et étanche aux fluides (8).

13. Système de seringue à deux chambres selon l'une quelconque des revendications 1 à 12, **caractérisé en ce que** la membrane perméable aux gaz et étanche aux fluides (8) est une membrane de filtre stérile.

14. Système de seringue à deux chambres selon la revendication 13, dans lequel la membrane de filtre stérile présente une taille de pores inférieure ou égale à 0,2 µm.

15. Système de seringue à deux chambres selon l'une quelconque des revendications 1 à 14, **caractérisé en ce que** la chambre distale A contient un composant solide et la chambre proximale B contient un composant liquide.

16. Système de seringue à deux chambres selon l'une quelconque des revendications 1 à 14, **caractérisé en ce que** les deux chambres A et B contiennent un composant liquide.

17. Dispositif avec un système de seringue à deux chambres selon l'une quelconque des revendications 1 à 16 et un emballage pour le système de seringue à deux chambres, **caractérisé en ce que** l'emballage est fabriqué en un matériau en feuille étanche aux gaz.

18. Dispositif selon la revendication 17, **caractérisé en ce que** le matériau d'emballage est une feuille d'aluminium, une feuille d'aluminium doublée de matière plastique ou une feuille de matière plastique revêtue d'aluminium.

19. Dispositif d'application comportant un système de seringue à deux chambres selon l'une quelconque des revendications 1 à 16, une aiguille à monter sur l'extrémité distale de l'élément de cylindre (2) et en option une ou plusieurs compresses de désinfection.
